# EUROPEAN PATENT APPLICATION

(11) **EP 1 918 281 A1**
(43) Date of publication of application: **07.05.2008**
(21) Application number: 06384017.7
(22) Date of filing: 02.11.2006
(51) Int. Cl.: C07D 211/58, C07D 401/12, A61K 31/4468, A61P 3/00

(54) **Phenylamino-substituted piperidine compounds, their preparation and use as medicaments**

(71) Applicant: Laboratorios del Dr. Esteve S.A., 08041 Barcelona (ES)
(72) Inventor: GarcÍa-López, Mónica, 08018 Barcelona (ES); Mas-Prio, Josep, 08191 Rubi(Barcelona) (ES); Torrens-Jover, Antonio, 08221 Tarressa (ES)
(74) Representative: Peters, Hajo

(57) **Abstract**

The present invention relates to phenylamino-substituted piperidine compounds of general formula (I), methods for their preparation, medicaments comprising these compounds as well as their use for the preparation of a medicament for the treatment of humans or animals.

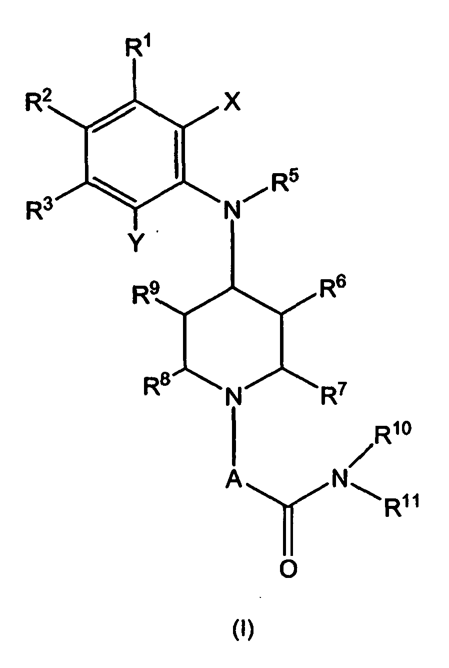

## Description

The present invention relates to phenylamino-substituted piperidine compounds of general formula (I), methods for their preparation, medicaments comprising these compounds as well as their use for the preparation of a medicament for the treatment of humans or animals.

Neuropeptide Y (NPY), first isolated in porcine brain extracts (Tatemoto et. al. Nature 1982, 296, 659), is a 36-aminoacid peptide belonging to the family of pancreatic polypeptides, and is one of the most abundant peptides in the brain and in the central nervous system. In addition, NPY is also distributed in several parts of the peripheral nervous system.

Several studies suggest a significant role of NPY in food ingestion regulation and particularly in food dysfunctions like obesity, anorexia and bulimia. Specifically, NPY is a powerful stimulant of food ingestion. Thus, appetite is significantly increased when NPY is injected directly into the CNS of satiated mice (Clark J. T. et. al. Endocrinology 1984, 115, 427; Levine A. S. et. al. Peptides 1984, 5, 1025; Stanley B. G. et. al. Life Sci. 1984, 35, 2635; Stanley B. G. et. al. Proc. Nat. Acad. Sci. USA 1985, 82, 3940). On the other hand, NPY may play a role in cognitive function regulation, e. g. memory (Flood J. F. et. al. Brain Res. 1987, 421, 280; Redrobe J. P. et. Al. Brain Res. 1999, 848, 153), and be active in anxiety (Heilig M. et. al. Reg. Peptides 1992, 41, 61) and depression (Heilig M. et. al. Eur. J. Pharmacol. 1988, 147, 465) processes.

NPY is also distributed in the peripheral system. Some studies suggest that it might be involved in hypertensive (Michel M. C: et. al. J. Hypertens. 1995, 13, 385), and analgesic (Gehlert D. R. Life Sci. 1994, 55, 551) processes, among others.

The endogenous proteins that constitute NPY-binding receptors have been widely studied. Several have been cloned and expressed. At present, six different receptor subtypes, named Y1 to Y6, are recognized (Hispkind P. A. et. al. Annu. Rep. Med. Chem. 1996, 31, 1; Grundemar L. et. al. TIPS Reviews., 15, 153, 1994). Each NPY receptor subtype is generally associated to a different biological activity. For example, Y2 receptor is involved in the induction of convulsions in rats (Dumont Y. et. al. Brit. J. Pharmacol. 2000, 129, 1075).

The most recently identified receptor is Y5 (Hu et. al. J. Biol. Chem. 1996, 271, 26315). There is evidence that Y5 receptor has a unique pharmacological profile related to food ingestion as compared to the other receptor subtypes. The fact that [D-Trp³²]NPY peptide, a selective Y5-receptor agonist with no affinity for Y1 receptor, stimulates food ingestion in rats (Gerald C. et. al. Nature, 1996, 382, 168), supports the hypothesis that Y5 receptor is related to exaggerated food consumption. Consequently, compounds having an affinity to the Y5 receptor should be effective to inhibit food ingestion and very useful to control diseases like obesity or other disorders of food ingestion (food intake), such as anorexia, bulimia, cachexia or type II diabetes. Moreover, it has been suggested that such compounds are useful to control diseases such as arthritis or epilepsy.

Several NPY5 non-peptidic antagonists have been described. Thus, 2-aminoquinazoline derivatives [PCT Int. Appl. WO 9720823, 1997 (Novartis AG)], sulfonamides [PCT Int. Appl. WO 9719682, 1997 (Synaptic Pharmaceutical Corp.)],
pyrazoles [PCT Int. Appl. WO 9824768, 1998 (Banyu Pharmaceutical Co., Ltd)], aminopyridines [PCT Int. Appl. WO 9840356, 1998 (Banyu Pharmaceutical Co., Ltd)], N-aralkyl-2-tetralinamines [PCT Int. Appl. WO 0020376, 2000 (Ortho McNeil Pharmaceutical Inc.)], several amides [PCT Int. Appl. WO 9835957, 1998 (Bayer Corp.)], pyridine and pyrimidine derivatives [PCT Int. Appl. WO 9940091, 1999 ( Amgen Inc.)], carbazoles [PCT Int. Appl. WO 0107409, 2001 (Astra Zeneca AB.)], and spirolsoquinolinones [PCT Int. Appl. WO 0113917, 2001 (Bristol-Myers Squibb Co.)], have been prepared.

Thus, it was an object of the present invention to provide novel compounds that are suitable in particular as active substances in medicaments, preferably in medicaments for the regulation of neuropeptide Y receptors, particularly preferably of neuropeptide Y 5 (NPY5) receptor, for the regulation of food ingestion (food intake), preferably for the prophylaxis and/or treatment of disorders of food ingestion, such as obesity, anorexia, cachexia, bulimia or type II (non insulin dependent) diabetes, for the prophylaxis and/or treatment of disorders of the peripheral nervous system, disorders of the central nervous system, anxiety, depression, cognitive disorders, preferably memory disorders, cardiovascular diseases, pain, epilepsy, arthritis, hypertensive syndrom, inflammatory diseases, immune diseases and other NPY5 mediated disorders in animals and mammals, including man.

Said object was achieved by providing phenylamino-substituted piperidine compounds of general formula (I), 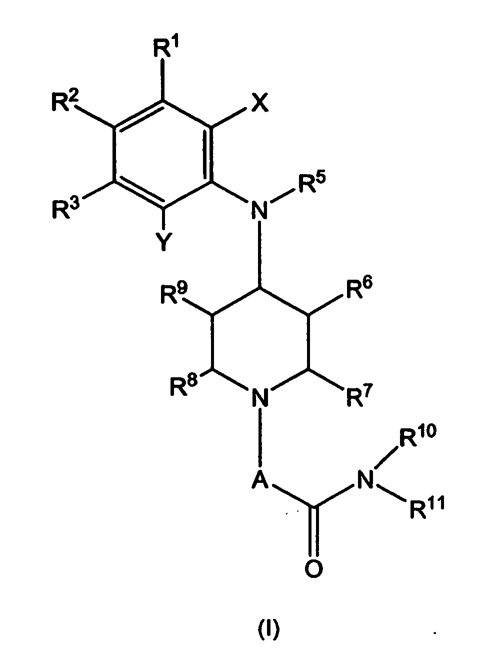 wherein
X and Y are each independently selected from the group consisting of hydrogen, halogen, a nitro, -OR¹², -OC(=O)R¹³, -SR¹⁴, -SOR¹⁴, -SO₂R¹⁴, -NH-SO₂R¹⁴, -SO₂NH₂ and -NR¹⁵R¹⁶ moiety;
R¹, R², R³, are each independently selected from the group consisting of hydrogen; halogen; an unbranched or branched, saturated or unsaturated, optionally at least mono-substituted aliphatic radical; a nitro, cyano, -OR¹², -OC(=O)R¹³, -SR¹⁴, -SOR¹⁴, - SO₂R¹⁴, -NH-SO₂R¹⁴, -SO₂NH₂ and -NR¹⁵R¹⁶ moiety,
R⁵ represents hydrogen; an unbranched or branched, saturated or unsaturated, optionally at least mono-substituted aliphatic radical; or a group 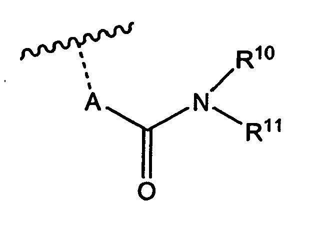 R⁶, R⁷, R⁸, R⁹ are each independently selected from the group consisting of hydrogen; an unbranched or branched, saturated or unsaturated, optionally at least mono-substituted aliphatic radical; a cyano or a COOR¹⁷ moiety,
A represents a bridge member -CHR¹⁸- or -CHR¹⁸-CH₂-;
R¹⁰ represents hydrogen; an unbranched or branched, saturated or unsaturated, optionally at least mono-substituted aliphatic radical;
R¹¹ represents an unbranched or branched, saturated or unsaturated, optionally at least mono-substituted aliphatic radical; a saturated or unsaturated, optionally at least mono-substituted cycloaliphatic radical, which may be bonded via an optionally at least mono-substituted alkylene group and/or may be condensed with an optionally at least mono-substituted mono- or polycyclic ringsystem, and which optionally may be bonded to another mono-or polycyclic ringsystem through either a direct bond, an optionally at least mono-substituted alkylene group, an ether bond or a keto-group; or an optionally at least mono substituted aryl radical, which may be bonded via an optionally at least mono-substituted alkylene group and/or may be condensed with an optionally at least mono-substituted mono- or polycyclic ringsystem, which optionally may be bonded to another mono-or polycyclic ringsystem through either a direct bond, an optionally at least mono-substituted alkylene group, an ether bond or a keto-group; or an optionally at least mono substituted heterocyclyl radical, which may be bonded via an optionally at least mono-substituted alkylene group and/or may be condensed with an optionally at least mono-substituted mono- or polycyclic ringsystem, and which optionally may be bonded to another mono-or polycyclic ringsystem through either a direct bond, an optionally at least mono-substituted alkylene group, an ether bond or a keto-group; or
R¹⁰ and R¹¹ together with the bridging nitrogen atom form an optionally at least mono-substituted, saturated, unsaturated or aromatic heterocyclic ring that may contain at least one further heteroatom as a ring member and/or may be condensed with an optionally at least mono-substituted mono- or polycyclic ringsystem;
R¹² , R¹³, R¹⁴ are hydrogen; or an unbranched or branched, saturated or unsaturated, optionally at least mono-substituted aliphatic radical;
R¹⁵ and R¹⁶ each are independently selected from the group consisting of hydrogen; or an unbranched or branched, saturated or unsaturated, optionally at least mono-substituted aliphatic radical;
R¹⁷ represents hydrogen; or an unbranched or branched, saturated or unsaturated, optionally at least mono-substituted aliphatic radical;
R¹⁸ represents hydrogen; or an unbranched or branched, saturated or unsaturated, optionally at least mono-substituted aliphatic radical;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

In regards to compounds of general formula (I) it is preferred if the following proviso applies:
• If either or both of X or Y is OR¹² with R¹² being hydrogen then R¹¹ may not be 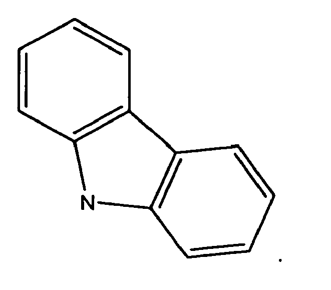 In regards to compounds of general formula (I) it is also preferred if the following proviso applies:
• R¹¹ may not be indole.

In regards to compounds of general formula (I) it is also preferred if the following proviso applies: 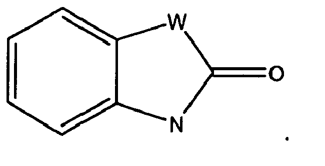
• R¹¹ may not be

A "mono- or polycyclic ring-system" according to the present invention means a mono- or polycyclic hydrocarbon ring-system that may be saturated, unsaturated or aromatic. If the ring system is polycyclic, each of its different rings may show a different degree of saturation, i.e. it may be saturated, unsaturated or aromatic. Optionally each of the rings of the mono- or polycyclic ring system may contain one or more heteroatoms as ring members, which may be identical or different and which can preferably be selected from the group consisting of N, O, S and P, more preferably be selected from the group consisting of N, O and S. Preferably the polycyclic ring-system may comprise two rings that are condensed. The rings of the mono- or polycyclic ring-sytem are preferably 5- or 6-membered.

An "aryl radical" or group is understood as meaning ring systems with at least one aromatic ring but without heteroatoms even in only one of the rings. Examples are phenyl, naphthyl, fluoranthenyl, fluorenyl, tetralinyl or indanyl, in particular 9H-fluorenyl or anthracenyl radicals, which can be unsubstituted or monosubstituted or polysubstituted.

In the context of this invention "cycloalkyl radical" or group is understood as meaning saturated and unsaturated (but not aromatic) cyclic hydrocarbons (without a heteroatom in the ring), which can be unsubstituted or mono- or polysubstituted. Furthermore, C₃₋₄-cycloalkyl represents C₃- or C₄-cycloalkyl, C₃₋₅-cycloalkyl represents C₃-, C₄- or C₅-cycloalkyl, C₃₋₆-cycloalkyl represents C₃-, C₄-, C₅- or C₆-cycloalkyl, C₃₋₇-cycloalkyl represents C₃-, C₄-, C₅-, C₆- or C₇-cycloalkyl, C₃₋₈-cycloalkyl represents C₃-, C₄-, C₅-, C₆-, C₇ or C₈-cycloalkyl, C₄-5-cycloalkyl represents C₄- or C₅-cycloalkyl, C₄₋₆-cycloalkyl represents C₄-, C₅- or C₆-cycloalkyl, C₄₋₇-cycloalkyl represents C₄-, C₅-, C₆- or C₇-cycloalkyl, C₄₋₈-cycloalkyl represents C₄-, C₅-, C₆- C₇- or C₈-cycloalkyl C₅₋₃-cycloalkyl represents C₅- or C₆-cycloalkyl and C₅₋₇-cycloalkyl represents C₅-, C₆- or C₇-cycloalkyl. However, mono- or polyunsaturated, preferably monounsaturated, cycloalkyls also in particular fall under the term cycloalkyl as long as the cycloalkyl is not an aromatic system. The cycloalkyl radicals are preferably cyclopropyl, 2-methylcyclopropyl, cyclopropylmethyl, cyclobutyl, cyclopentyl, cyclopentylmethyl, cyclohexyl, cycloheptyl, cyclooctyl, and also adamantly.

A "heterocyclyl radical" or group is understood as meaning heterocyclic ring systems, saturated or unsaturated ring which contains one or more heteroatoms from the group consisting of nitrogen, oxygen and/or sulfur in the ring and can also be mono- or polysubstituted. Examples which may be mentioned from the group of heterocyclyls are furan, benzofuran, thiophene, benzothiophene, pyrrole, pyridine, pyrimidine, pyrazine, quinoline, isoquinoline, phthalazine, benzo-1,2,5-thiadiazole, benzothiazole, indole, benzotriazole, benzodioxolane, benzodioxane, carbazole and quinazoline.

In connection with mono- or polycyclic ring-system, aryl radical, cycloalkyl radical, or heterocyclyl radical, "substituted" is understood - unless defined otherwise - as meaning replacement of at least one hydrogen radical on the ring-system of the mono- or polycyclic ring-system, the aryl radical, the cycloalkyl radical, or the heterocyclyl radical by OH, SH, =O, halogen (F, Cl, Br, I), CN, NO₂, COOH; NRₓR_{y}, with Rₓ and R_{y} independently being either H or a saturated or unsaturated, linear or branched, substituted or unsubstituted C₁₋₆-alkyl; by a saturated or unsaturated, linear or branched, substituted or unsubstituted C₁₋₆-alkyl; a saturated or unsaturated, linear or branched, substituted or unsubstituted -O-C₁₋₆-alkyl (alkoxy); a saturated or unsaturated, linear or branched, substituted or unsubstituted -S-C₁₋₆-alkyl; a saturated or unsaturated, linear or branched, substituted or unsubstituted -C(O)-C₁₋₆-alkyl; a saturated or unsaturated, linear or branched, substituted or unsubstituted -C(O)-O-C₁₋₆-alkyl; a substituted or unsubstituted phenyl.

In connection with aryl radical, cycloalkyl radical, or heterocyclyl radical, "condensed with" is understood as meaning that the ring-system of the aryl radical, the cycloalkyl radical, or the heterocyclyl radical is sharing two atoms (one) of its ring(s) with a ring of the mono- or polycyclic ring-system it is condensed with.

Aliphatic radicals/groups, as referred to in the present invention, are optionally mono- or polysubstituted and may be branched or unbranched, saturated or unsaturated. Aliphatic radicals, as defined in the present invention, include alkyl, alkenyl and alkinyl radicals. Unsaturated aliphatic radicals, as defined in the present invention, include alkenyl and alkinyl radicals. Preferred aliphatic radicals according to the present invention include but are not restricted to methyl, ethyl, vinyl (ethenyl), ethinyl, propyl, n-propyl, isopropyl, allyl (2-propenyl), 1-propinyl, methylethyl, butyl, n-butyl, iso-butyl, sec-butyl, tert-butyl butenyl, butinyl, 1-methylpropyl, 2-methylpropyl, 1,1-dimethylethyl, pentyl, n-pentyl, 1,1-dimethylpropyl, 1,2-dimethylpropyl, 2,2-dimethylpropyl, hexyl, 1-methylpentyl, n-heptyl, n-octyl, n-nonyl and n-decyl.

In the context of this invention, alkyl radical or group is understood as meaning saturated and unsaturated, linear or branched hydrocarbons, which can be unsubstituted or mono- or polysubstituted. Thus unsaturated alkyl is understood to encompass alkenyl and alkinyl groups, like e.g. -CH=CH-CH₃ or -C=C-CH₃, while saturated alkyl encompasses e.g. -CH₃ and -CH₂-CH₃. In these radicals, C₁₋₂-alkyl represents C₁- or C₂-alkyl, C₁₋₃-alkyl represents C₁-, C₂- or C₃-alkyl, C₁₋₄-alkyl represents C₁-, C₂-, C₃- or C₄-alkyl, C₁₋₅-alkyl represents C₁-, C₂-, C₃-, C₄-, or C₅-alkyl, C₁₋₆-alkyl represents C₁-, C₂-, C₃-, C₄-, C₅- or C₆-alkyl, C₁₋₇-alkyl represents C₁-, C₂-, C₃-, C₄-, C₅-, C₆- or C₇-alkyl, C₁₋₈-alkyl represents C₁-, C₂-, C₃-, C₄-, C₅-, C₆-, C₇- or C₈-alkyl, C₁₋₁₀-alkyl represents C₁-, C₂-, C₃-, C₄-, C₅-, C₆-, C₇-, C₈-, C₉- or C₁₀-alkyl and C₁₋₁₈-alkyl represents C₁-, C₂-, C₃-, C₄-, C₆-, C₆-, C₇-, C₈-, C₉-, C₁₀-, C₁₁-, C₁₂-, C₁₃-, C₁₄-, C₁₅-, C₁₆-. C₁₇- or C₁₈-alkyl. The alkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, if substituted also CHF₂, CF₃ or CH₂OH etc.

In connection with alkyl or aliphatic radical or group- unless defined otherwise - the term "substituted" in the context of this invention is understood as meaning replacement of at least one hydrogen radical by F, Cl, Br, I, NH₂, SH or OH; "polysubstituted" radicals being understood as meaning that the replacement takes effect both on different and on the same atoms several times with the same or different substituents, for example three times on the same C atom, as in the case of CF₃, or at different places, as in the case of e.g. -CH(OH)-CH=CH-CHCl₂.

The term "alkylene" is understood as meaning a divalent alkyl group like -CH₂- or -CH₂-CH₂-, with (CH₂)₃₋₆ being understood as meaning -CH₂-CH₂-CH₂-, -CH₂-CH₂-CH₂-CH₂-, -CH₂-CH₂-CH₂-CH₂-CH₂- and -CH₂-CH₂-CH₂-CH₂-CH₂-CH₂-, (CH₂)₁₋₄ is to be understood as meaning - CH₂-, -CH₂-CH₂-, -CH₂-CH₂-CH₂- and -CH₂-CH₂-CH₂-CH₂-, (CH₂)₄₋₅ is to be understood as meaning -CH₂-CH₂-CH₂-CH₂- and -CH₂-CH₂-CH₂-CH₂CH₂-, etc.

The term "salt" is to be understood as meaning any form of the active compound used according to the invention in which it assumes an ionic form or is charged and is coupled with a counter-ion (a cation or anion) or is in solution. By this are also to be understood complexes of the active compound with other molecules and ions, in particular complexes which are complexed via ionic interactions.

The term "physiologically acceptable salt" means in the context of this invention any salt that is physiologically tolerated (most of the time meaning not being toxic- especially not caused by the counter-ion) if used appropriately for a treatment especially if used on or applied to humans and/or mammals.

These physiologically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH4, but in particular (mono)- or (di)sodium, (mono)- or (di)potassium, magnesium or calcium salts.

These physiologically acceptable salts can also be formed with anions or acids in the context of this invention is understood as meaning salts of at least one of the compounds used according to the invention - usually protonated, for example on the nitrogen - as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of: hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

The compounds of the invention may be in crystalline form or either as free compounds or as solvates and it is intended that those forms are within the scope of the present invention. Methods of solvation are generally known within the art. Suitable solvates are pharmaceutically acceptable solvates. The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or ¹⁵N-enriched nitrogen are within the scope of this invention.

Any compound that is a prodrug of a compound of formula (I) is within the scope of the invention. The term "prodrug" is used in its broadest sense and encompasses those derivatives that are converted in vivo to the compounds of the invention. Such derivatives would readily occur to those skilled in the art, and include, depending on the functional groups present in the molecule and without limitation, the following derivatives of the present compounds: esters, amino acid esters, phosphate esters, metal salts sulfonate esters, carbamates, and amides. Examples of well known methods of producing a prodrug of a given acting compound are known to those skilled in the art and can be found e.g. in Krogsgaard-Larsen et al. "Textbook of Drug design and Discovery" Taylor & Francis (April 2002).

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I) or, or of its salts, solvates or prodrugs.

Particularly preferred are compounds of general formula (1), wherein X and Y are each independently selected from the group consisting of hydrogen, halogen, a nitro, or -OR¹² moiety; preferably X and Y are each independently selected from the group consisting of hydrogen, or -OR¹² moiety.

Also particularly preferred are compounds of general formula (I), wherein R¹, R², R³, are each independently selected from the group consisting of hydrogen; halogen; an unbranched or branched, saturated or unsaturated, optionally at least mono-substituted aliphatic radical; a nitro, -OR¹², -OC(=O)R¹³, and -NR¹⁵R¹⁶ moiety; preferably R¹, R², R³, are each independently selected from the group consisting of hydrogen; or -OR¹² moiety.

Also particularly preferred are compounds of general formula (I), wherein R¹², R¹³, R¹⁴ are hydrogen; or an optionally substituted branched or unbranched C₁₋₄-alkyl radical; preferably R¹², R¹³, R¹⁴ represent H, CH₃, CF₃, CH₂CH₃, CH₂CH₂CH₃, CH(CH₃)₂ or C(CH₃)₃.

Also particularly preferred are compounds of general formula (I), wherein R¹⁵ and R¹⁶ are each independently selected from the group consisting of H, or a C₁₋₄-alkyl radical, preferably selected from the group consisting of H, CH₃, and C₂H₅.

Also particularly preferred are compounds of general formula (I), wherein R⁵ represents H or a branched or unbranched C₁₋₃-alkyl radical, or a group 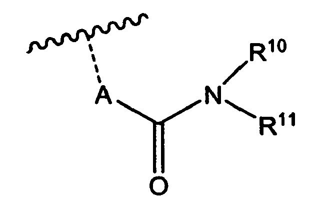 peferably R⁵ represents H, CH₃ or CH₂CH₃; or a group 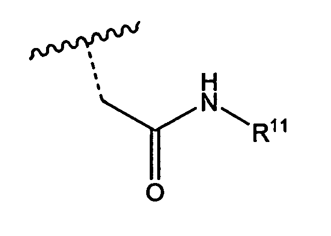 most preferably R⁵ represents H; or a group

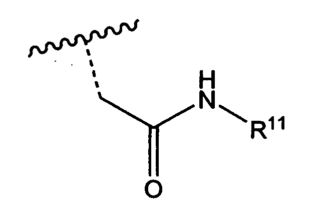

Also particularly preferred are compounds of general formula (I), wherein R⁶, R⁷, R⁸, R⁹ are each independently selected from the group consisting of H, a branched or unbranched C₁₋₃-alkyl radical, or a cyano-moiety; preferably from the group consisting of H, CH₃, CH₂CH₃ and a cyano-moiety, more preferably all represent H.

Also particularly preferred are compounds of general formula (I), wherein R¹⁷ represents H, or a C₁₋₄-alkyl radical; preferably R¹⁷ represents H, CH₃ or C₂H₅.

Also particularly preferred are compounds of general formula (I), wherein R¹⁸ represents H, or a C₁₋₄-alkyl radical or a phenyl radical, preferably R¹⁸ represents H, or CH₃, more preferably R¹⁸ represents H.

Also particularly preferred are compounds of general formula (I), wherein R¹⁰ represents hydrogen or a branched or unbranched C₁₋₄-alkyl radical; preferably R¹⁰ represents H, CH₃, CH₂CH₃, CH₂CH₂CH₃, CH(CH₃)₂ or C(CH₃)₃; more preferably R¹⁰ represents H.

Also particularly preferred are compounds of general formula (I), wherein R¹¹ is selected from the group consisting of an optionally at least mono substituted aryl radical, which may be bonded via an optionally at least mono-substituted alkylene group and/or may be condensed with an optionally at least mono-substituted mono- or polycyclic ringsystem, which optionally may be bonded to another mono-or polycyclic ringsystem through either a direct bond, an optionally at least mono-substituted alkylene group, an ether bond or a keto-group; or an optionally at least mono substituted heterocyclyl radical, which may be bonded via an optionally at least mono-substituted alkylene group and/or may be condensed with an optionally at least mono-substituted mono- or polycyclic ringsystem, and which optionally may be bonded to another mono-or polycyclic ringsystem through either a direct bond, an optionally at least mono-substituted alkylene group, an ether bond or a keto-group;
preferably R¹¹ is selected from the group consisting of an optionally at least mono substituted phenyl or pyridine radical, which may be condensed with an optionally at least mono-substituted mono- or polycyclic ringsystem, and which optionally may be bonded to another mono-or polycyclic ringsystem through either a direct bond, an optionally at least mono-substituted alkylene group, an ether bond or a keto-group;
more preferably R¹¹ is selected from the group consisting of 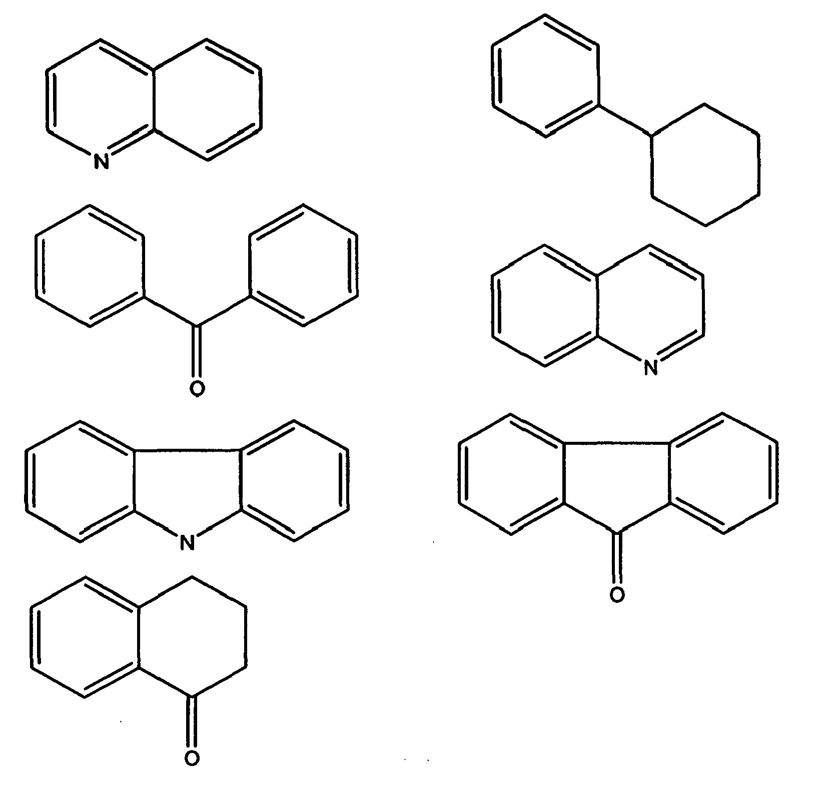 optionally at least monosubstituted.

Also particularly preferred are compounds of general formula (I), wherein R¹⁰ and R¹¹ together with the bridging nitrogen atom form a saturated, 6-membered heterocyclic ring, which is at least mono-substituted with a methyl radical and/or condensed with an unsubstituted or at least mono-substituted phenyl- or cyclohexyl-radical, said phenyl- or cyclohexyl-radical preferably being at least mono-substituted with F and/or OCH₃.

Highly preferred are also compounds of general formula (Ia) 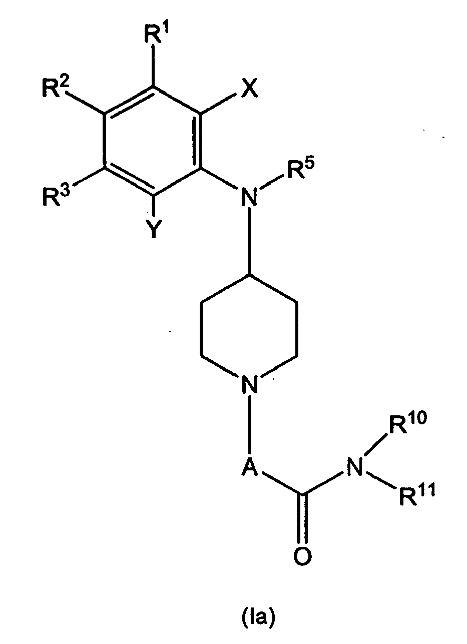 wherein
X and Y are each independently selected from the group consisting of hydrogen, halogen, a nitro, or -OR¹² moiety;
R¹, R², R³, are each independently selected from the group consisting of hydrogen; halogen; an unbranched or branched, saturated or unsaturated, optionally at least mono-substituted aliphatic radical; a nitro, -OR¹², -OC(=O)R¹³, and -NR¹⁵R¹⁶ moiety,
R⁵ represents hydrogen; an unbranched or branched, saturated or unsaturated, optionally at least mono-substituted aliphatic radical; or a group 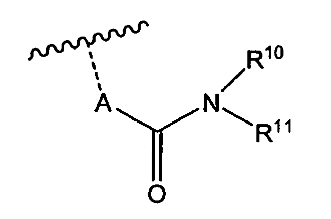 A represents a bridge member -CH₂- or -CH₂-CH₂-
R¹⁰ represents hydrogen; an unbranched or branched, saturated or unsaturated, optionally at least mono-substituted aliphatic radical;
R¹¹ represents an unbranched or branched, saturated or unsaturated, optionally at least mono-substituted aliphatic radical; a saturated or unsaturated, optionally at least mono-substituted cycloaliphatic radical, which may be bonded via an optionally at least mono-substituted alkylene group and/or may be condensed with an optionally at least mono-substituted mono- or polycyclic ringsystem, and which optionally may be bonded to another mono-or polycyclic ringsystem through either a direct bond, an optionally at least mono-substituted alkylene group, an ether bond or a keto-group; or an optionally at least mono substituted aryl radical, which may be bonded via an optionally at least mono-substituted alkylene group and/or may be condensed with an optionally at least mono-substituted mono- or polycyclic ringsystem, which optionally may be bonded to another mono-or polycyclic ringsystem through either a direct bond, an optionally at least mono-substituted alkylene group, an ether bond or a keto-group; or an optionally at least mono substituted heterocyclyl radical, which may be bonded via an optionally at least mono-substituted alkylene group and/or may be condensed with an optionally at least mono-substituted mono- or polycyclic ringsystem, and which optionally may be bonded to another mono-or polycyclic ringsystem through either a direct bond, an optionally at least mono-substituted alkylene group, an ether bond or a keto-group; or
R¹⁰ and R¹¹ together with the bridging nitrogen atom form an optionally at least mono-substituted, saturated, unsaturated or aromatic heterocyclic ring that may contain at least one further heteroatom as a ring member and/or may be condensed with an optionally at least mono-substituted mono- or polycyclic ringsystem;
R¹², R¹³ are hydrogen; or an unbranched or branched, saturated or unsaturated, optionally at least mono-substituted aliphatic radical;
R¹⁵ and R¹⁶ each are independently selected from the group consisting of hydrogen; or an unbranched or branched, saturated or unsaturated, optionally at least mono-substituted aliphatic radical;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

In regards to compounds of general formula (Ia) it is preferred if the following proviso applies:
• If either or both of X or Y is OR¹² with R¹² being hydrogen then R¹¹ may not be

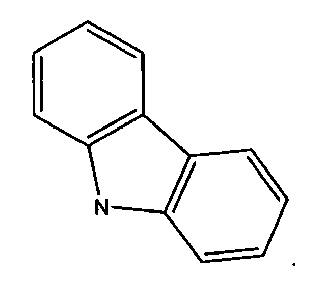

In regards to compounds of general formula (Ia) it is also preferred if the following proviso applies:
• R¹¹ may not be indole.

In regards to compounds of general formula (Ia) it is also preferred if the following proviso applies:
• R¹¹ may not be

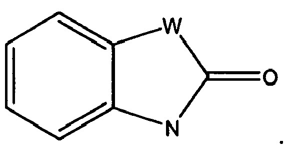

Also particularly preferred are compounds of general formula (Ia), wherein X and Y are each independently selected from the group consisting of hydrogen, or -OR¹² moiety.

Also particularly preferred are compounds of general formula (Ia), wherein R¹, R², R³, are each independently selected from the group consisting of hydrogen; F, Cl, Br; an optionally substituted branched or unbranched C₁₋₄-alkyl radical; an -OR¹² moiety; preferably R¹, R², R³, are each independently selected from the group consisting of hydrogen; or -OR¹² moiety;

Also particularly preferred are compounds of general formula (Ia), wherein R¹², R¹³ are hydrogen; or an optionally substituted branched or unbranched C₁₋₄-alkyl radical; preferably R¹², R¹³ represent H, CH₃, CF₃, CH₂CH₃, CH₂CH₂CH₃, CH(CH₃)₂ or C(CH₃)₃.

Also particularly preferred are compounds of general formula (Ia), wherein R¹⁵ and R¹⁶ are each independently selected from the group consisting of H, or a C₁₋₄-alkyl radical, preferably selected from the group consisting of H, CH₃, and C₂H₅.

Also particularly preferred are compounds of general formula (Ia), wherein R⁵ represents H or a branched or unbranched C₁₋₃-alkyl radical, or a group 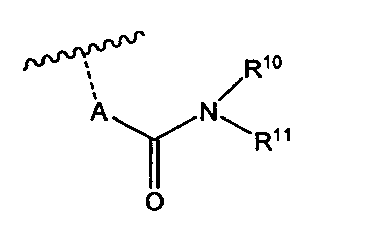 peferably R⁵ represents H, CH₃ or CH₂CH₃; or a group 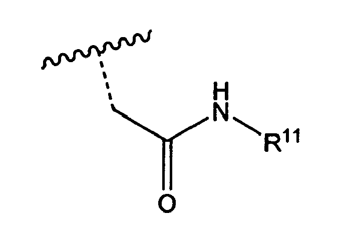 most preferably R⁵ represents H; or a group

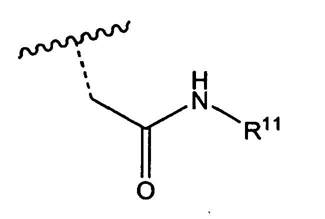

Also particularly preferred are compounds of general formula (1a), wherein R¹⁰ represents hydrogen or a branched or unbranched C₁₋₄-alkyl radical; preferably R¹⁰ represents H, CH₃, CH₂CH₃, CH₂CH₂CH₃, CH(CH₃)₂ or C(CH₃)₃; more preferably R¹⁰ represents H.

Also particularly preferred are compounds of general formula (1a), wherein R¹¹ is selected from the group consisting of an optionally at least mono substituted aryl radical, which may be bonded via an optionally at least mono-substituted alkylene group and/or may be condensed with an optionally at least mono-substituted mono- or polycyclic ringsystem, which optionally may be bonded to another mono-or polycyclic ringsystem through either a direct bond, an optionally at least mono-substituted alkylene group, an ether bond or a keto-group; or an optionally at least mono substituted heterocyclyl radical, which may be bonded via an optionally at least mono-substituted alkylene group and/or may be condensed with an optionally at least mono-substituted mono- or polycyclic ringsystem, and which optionally may be bonded to another mono-or polycyclic ringsystem through either a direct bond, an optionally at least mono-substituted alkylene group, an ether bond or a keto-group;
preferably R¹¹ is selected from the group consisting of an optionally at least mono substituted phenyl or pyridine radical, which may be condensed with an optionally at least mono-substituted mono- or polycyclic ringsystem, and which optionally may be bonded to another mono-or polycyclic ringsystem through either a direct bond, an optionally at least mono-substituted alkylene group, an ether bond or a keto-group;
more preferably R¹¹ is selected from the group consisting of 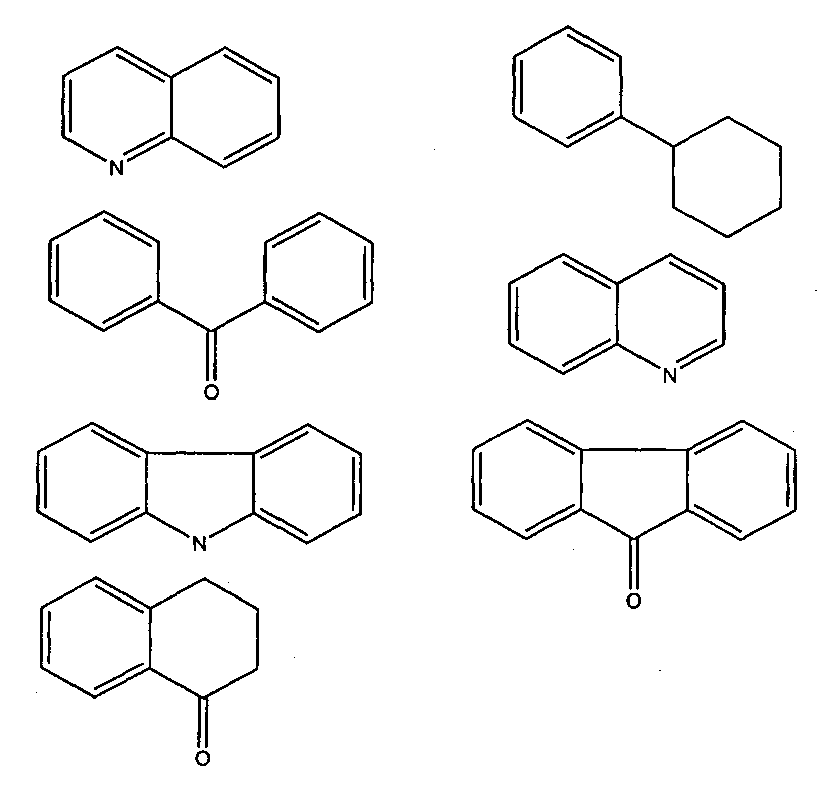 optionally at least monosubstituted.

Highly preferred are also compounds of general formula (Ib) 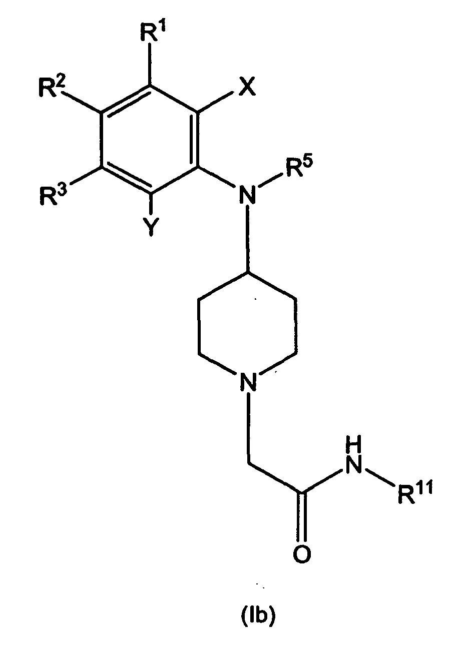 wherein
X and Y are each independently selected from the group consisting of hydrogen, or - OR¹² moiety;
R¹, R², R³, are each independently selected from the group consisting of hydrogen; or -OR¹² moiety,
R⁵ represents hydrogen; or a group 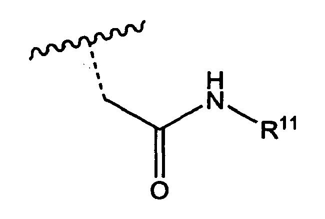 R¹¹ represents an unbranched or branched, saturated or unsaturated, optionally at least mono-substituted aliphatic radical; a saturated or unsaturated, optionally at least mono-substituted cycloaliphatic radical, which may be bonded via an optionally at least mono-substituted alkylene group and/or may be condensed with an optionally at least mono-substituted mono- or polycyclic ringsystem, and which optionally may be bonded to another mono-or polycyclic ringsystem through either a direct bond, an optionally at least mono-substituted alkylene group, an ether bond or a keto-group; or an optionally at least mono substituted aryl radical, which may be bonded via an optionally at least mono-substituted alkylene group and/or may be condensed with an optionally at least mono-substituted mono- or polycyclic ringsystem, which optionally may be bonded to another mono-or polycyclic ringsystem through either a direct bond, an optionally at least mono-substituted alkylene group, an ether bond or a keto-group; or an optionally at least mono substituted heterocyclyl radical, which may be bonded via an optionally at least mono-substituted alkylene group and/or may be condensed with an optionally at least mono-substituted mono- or polycyclic ringsystem, and which optionally may be bonded to another mono-or polycyclic ringsystem through either a direct bond, an optionally at least mono-substituted alkylene group, an ether bond or a keto-group;
R¹² is hydrogen or CH₃, C₂H₅ or CF₃;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

In regards to compounds of general formula (Ib) it is preferred if the following proviso applies:
• If either or both of X or Y is OR¹² with R¹² being hydrogen then R¹¹ may not be

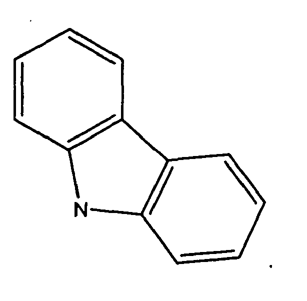

In regards to compounds of general formula (Ib) it is also preferred if the following proviso applies:
• R¹¹ may not be indole.

In regards to compounds of general formula (Ib) it is also preferred if the following proviso applies:
• R¹¹ may not be

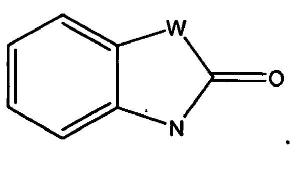

Also particularly preferred are compounds of general formula (Ib), wherein R¹, R², R³, X or Y are each independently selected from the group consisting of H, OH or OCH₃.

Also particularly preferred are compounds of general formula (Ib), wherein R¹², R¹³ are hydrogen; or an optionally substituted branched or unbranched C₁₋₄-alkyl radical; preferably R¹², R¹³ represent H, CH₃, CF₃, CH₂CH₃, CH₂CH₂CH₃, CH(CH₃)₂ or C(CH₃)₃.

Also particularly preferred are compounds of general formula (lb), wherein R⁵ represents H.

Also particularly preferred are compounds of general formula (lb), wherein R⁵ represents

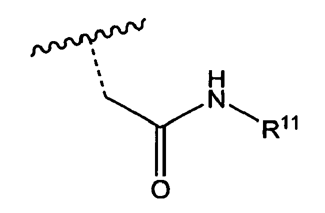

Also particularly preferred are compounds of general formula (Ib), wherein R¹¹ is selected from the group consisting of an optionally at least mono substituted aryl radical, which may be bonded via an optionally at least mono-substituted alkylene group and/or may be condensed with an optionally at least mono-substituted mono- or polycyclic ringsystem, which optionally may be bonded to another mono-or polycyclic ringsystem through either a direct bond, an optionally at least mono-substituted alkylene group, an ether bond or a keto-group; or an optionally at least mono substituted heterocyclyl radical, which may be bonded via an optionally at least mono-substituted alkylene group and/or may be condensed with an optionally at least mono-substituted mono- or polycyclic ringsystem, and which optionally may be bonded to another mono-or polycyclic ringsystem through either a direct bond, an optionally at least mono-substituted alkylene group, an ether bond or a keto-group;
preferably R¹¹ is selected from the group consisting of an optionally at least mono substituted phenyl or pyridine radical, which may be condensed with an optionally at least mono-substituted mono- or polycyclic ringsystem, and which optionally may be bonded to another mono-or polycyclic ringsystem through either a direct bond, an optionally at least mono-substituted alkylene group, an ether bond or a keto-group;
more preferably R¹¹ is selected from the group consisting of 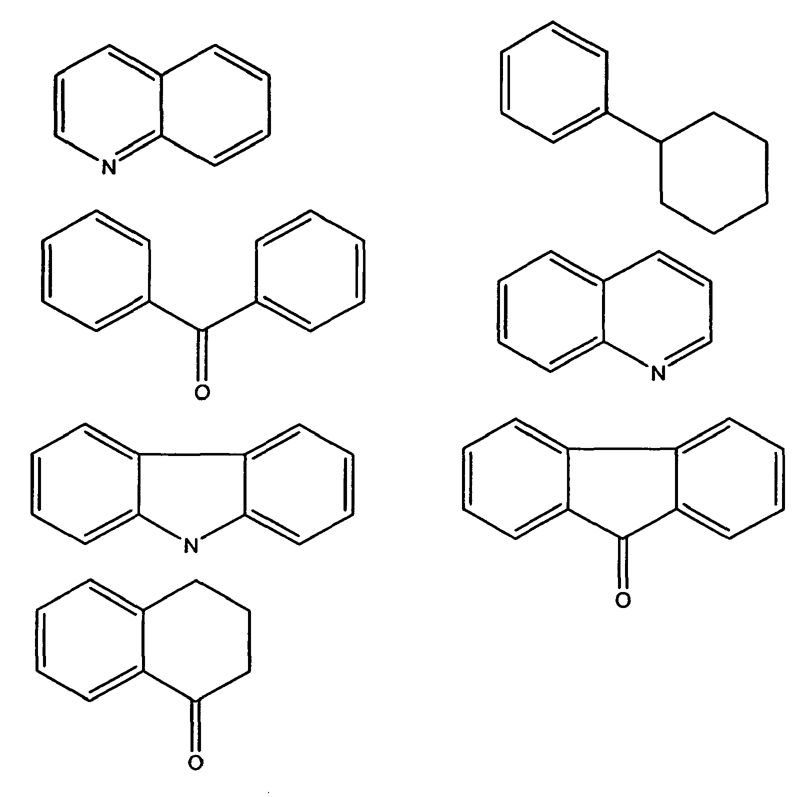 optionally at least monosubstituted.

Also preferred are compounds of general formula (I), (Ia) or (Ib) wherein R¹¹ is selected from the group consisting of 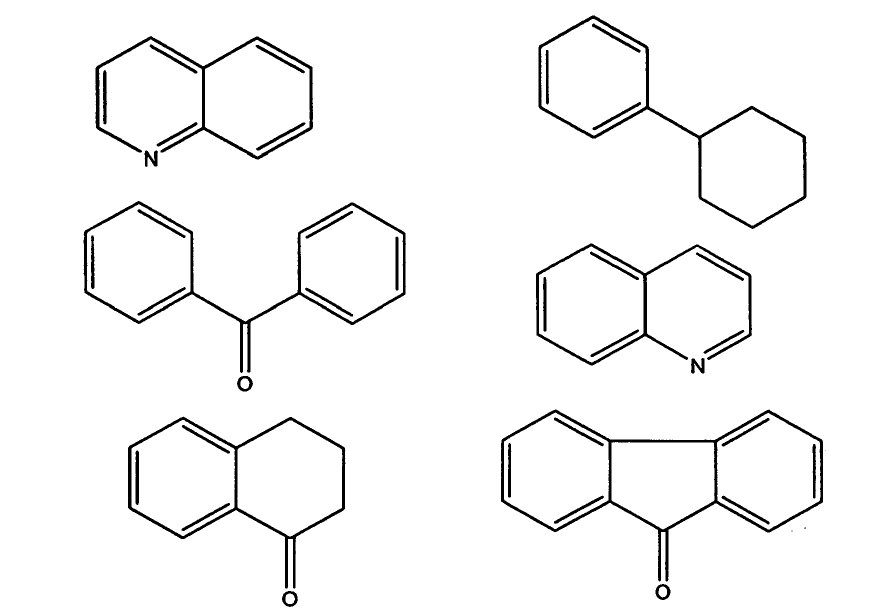 optionally at least monosubstituted.

Also preferred are compounds of general formula (I), (Ia) or (Ib) wherein R¹¹ is selected from the group consisting of 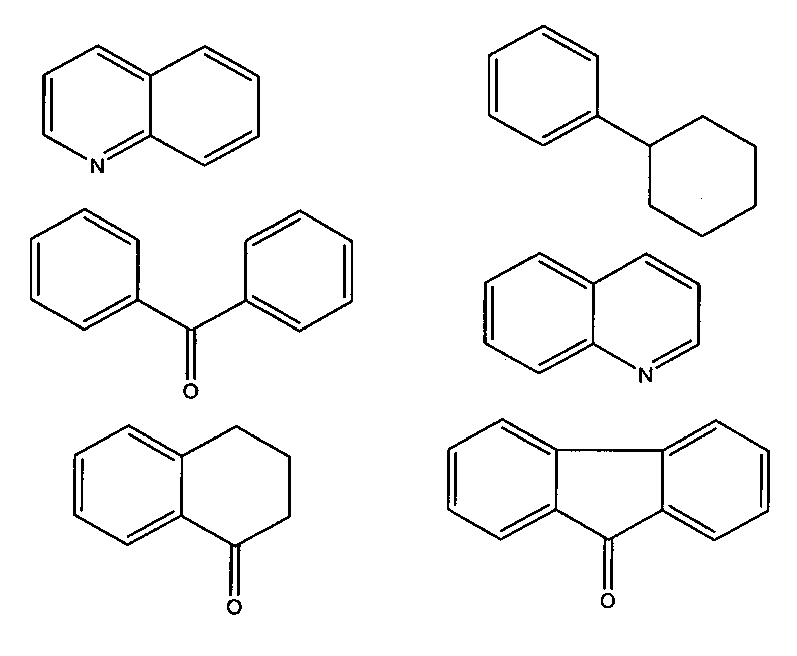 optionally at least monosubstituted;
while R¹ to R³, R⁵ to R¹⁰, A, X and Y are as given for a compound of general formula (I) above; or
while R¹ to R³, R⁵, R¹⁰, A, X and Y are as given for a compound of general formula (Ia) above; or
while R¹ to R³, R⁵, X and Y are as given for a compound of general formula (Ib) above.

Also particularly preferred are compounds of general formula (I), (1a) or (lb), wherein R¹, R², R³, X or Y are each independently selected from the group consisting of H, OH or OCH₃, and one of R¹, R², R³, X or Y is OH or OCH₃ or all of R¹, R², R³, X or Y are hydrogen;
more preferably wherein R¹, R², R³, X or Y are each independently selected from the group consisting of H, OH or OCH₃, and one of R¹, R², R³, X or Y is OH or OCH₃ and all others are hydrogen or all of R¹, R², R³, X or Y are hydrogen.

Also particularly preferred are compounds of general formula (I), (Ia) or (Ib), wherein R¹, R², R³, X or Y are each independently selected from the group consisting of H, OH or OCH₃, and one of R¹, R², R³, X or Y is OH or OCH₃ or all of R¹, R², R³, X or Y are hydrogen;
more preferably wherein R¹, R², R³, X or Y are each independently selected from the group consisting of H, OH or OCH₃, and one of R¹, R², R³, X or Y is OH or OCH₃ and all others are hydrogen or all of R¹, R², R³, X or Y are hydrogen;
while R⁵ to R¹¹ and A are as given for a compound of general formula (I) above; or while R⁵, R¹⁰, R¹¹ and A are as given for a compound of general formula (Ia) above; or while R⁵ and R¹¹ are as given for a compound of general formula (Ib) above.

Also particularly preferred are compounds of general formula (I), (Ia) or (Ib),
wherein R¹, R², R³, X or Y are each independently selected from the group consisting of H, OH or OCH₃;
preferably wherein R¹, R², R³, X or Y are each independently selected from the group consisting of H, OH or OCH₃, and one of R¹, R², R³, X or Y is OH or OCH₃ or all of R¹, R², R³, X or Y are hydrogen;
most preferably wherein R¹, R², R³, X or Y are each independently selected from the group consisting of H, OH or OCH₃, and one of R¹, R², R³, X or Y is OH or OCH₃ and all others are hydrogen or all of R¹, R², R³, X or Y are hydrogen;
and
wherein R¹¹ is selected from the group consisting of 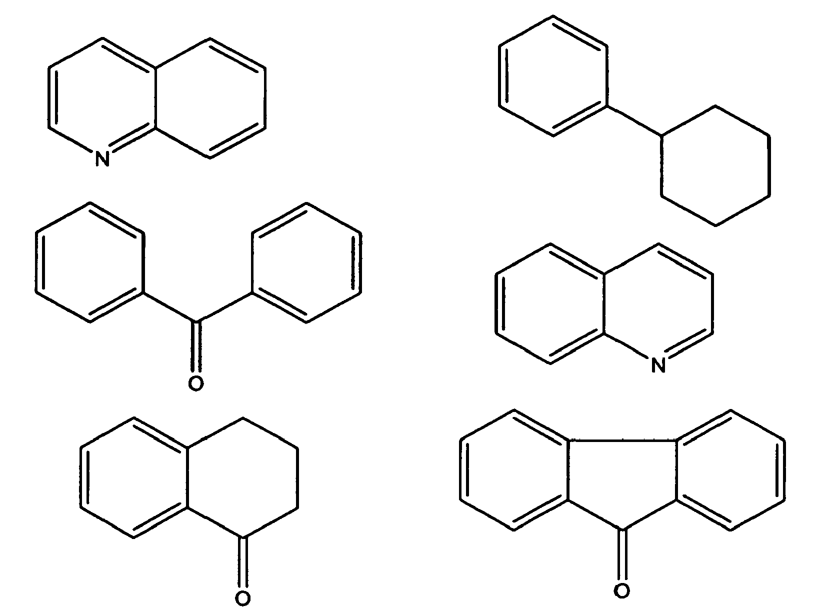 optionally at least monosubstituted;
while R⁵ to R¹⁰ and A are as given for a compound of general formula (I) above; or while R⁵, R¹⁰ and A are as given for a compound of general formula (Ia) above; or while R⁵ is as given for a compound of general formula (Ib) above.

Also particularly preferred are compounds of general formula (I), (Ia) or (Ib), wherein
X and Y are each independently selected from the group consisting of H or OCH₃; preferably wherein
X and Y are each independently selected from the group consisting of H or OCH₃, and R¹, R² and R³ are each independently selected from H, OH or OCH₃.

Also particularly preferred are compounds of general formula (I), (Ia) or (Ib), wherein
X and Y are each Independently selected from the group consisting of H or OCH₃;
while R¹ to R³, R⁶ to R¹¹ and A are as given for a compound of general formula (I) above; or
while R¹ to R³, R⁵, R¹⁰, R¹¹ and A are as given for a compound of general formula (1a) above; or
while R⁵ and R¹¹ are as given for a compound of general formula (Ib) above.

Also particularly preferred are compounds of general formula (I), (Ia) or (Ib), wherein
X and Y are each independently selected from the group consisting of H or OCH₃, and R¹, R² and R³ are each independently selected from H, OH or OCH₃;
while R⁵ to R¹¹,and A are as given for a compound of general formula (I) above; or while R⁵, R¹⁰ and R¹¹, and A are as given for a compound of general formula (1a) above; or
while R⁵ and R¹¹ are as given for a compound of general formula (Ib) above.

Also particularly preferred are compounds of general formula (I), (Ia) or (Ib), wherein R¹, R², R³, X or Y are all hydrogen.

Also particularly preferred are compounds of general formula (I), (Ia) or (Ib), wherein R¹, R², R³, X or Y are all hydrogen;
while R⁵ to R¹¹, and A are as given for a compound of general formula (I) above; or while R⁵, R¹⁰ and R¹¹, and A are as given for a compound of general formula (Ia) above; or
while R⁵ and R¹¹ are as given for a compound of general formula (Ib) above.

Also particularly preferred are compounds of general formula (I). (Ia) or (Ib), wherein
R¹, R², R³, X or Y are each independently selected from the group consisting of H, OH or OCH₃, and one of R¹, R², R³, X or Y is OH or OCH₃;
more preferably wherein R¹, R², R³, X or Y are each independently selected from the group consisting of H, OH or OCH₃, and one of R¹, R², R³ X or Y is OH or OCH₃ and all others are hydrogen.

Also particularly preferred are compounds of general formula (I), (Ia) or (Ib), wherein
R¹, R², R³, X or Y are each independently selected from the group consisting of H, OH or OCH₃, and one of R¹, R², R³, X or Y is OH or OCH₃;
more preferably wherein R¹, R², R³, X or Y are each independently selected from the group consisting of H, OH or OCH₃, and one of R¹, R², R³ X or Y is OH or OCH₃ and all others are hydrogen;
while R⁵ to R¹¹ and A are as given for a compound of general formula (I) above; or while R⁵, R¹⁰, R¹¹ and A are as given for a compound of general formula (1a) above; or while R⁵ and R¹¹ are as given for a compound of general formula (lb) above.

Most preferred are the following phenylamino-substituted piperidine compounds of general formula (I):
2-[4-(2-Hydroxy-phenylamino)-piperidin-1-yl]-N-quinolin-3-yl-acetamide
N-(4-Benzoyl-phenyl)-2-[4-(2-hydroxy-phenylamino)-piperidin-1-yl]-acetamide
N-(9-Ethyl-9H-carbazol-3-yl)-2-[4-(2-hydroxy-phenylamino)-piperidin-1-yl]-acetamide
N-(4-Cyclohexyl-phenyl)-2-[4-(2-hydroxy-phenylamino)-piperidin-1-yl]-acetamide
2-[4-(2-Hydroxy-phenylamino)-piperidin-1-yl]-N-quinolin-6-yl-acetamide
2-[4-(2-Hydroxy-phenylamino)-piperidin-1-yl]-N-(9-oxo-9H-fluoren-3-yl)-acetamide
N-(4-Acetyl-phenyl)-2-[4-(2-hydroxy-phenylamino)-piperidin-1-yl]-acetamide
N-(3-Acetyl-phenyl)-2-[4-(2-hydroxy-phenylamino)-piperidin-1-yl]-acetamide
2-[4-(2-Hydroxy-phenylamino)-piperidin-1-yl]-N-(5-oxo-5,6,7,8-tetrahydro-naphthalen-2-yl)-acetamide
N-(9-Ethyl-9H-carbazol-3-yl)-2-[4-(3-hydroxy-phenylamino)-piperidin-1-yl]-acetamide
2-[4-(3-Hydroxy-phenylamino)-piperidin-1-yl]-N-(9-oxo-9H-fluoren-3-yl)-acetamide
N-{4-[Ethyl-(2-hydroxy-ethyl)-amino]-phenyl}-2-[4-(3-hydroxy-phenylamino)-piperidin-1-yl]-acetamide
2-[4-(3-Hydroxy-phenylamino)-piperidin-1-yl]-N-quinolin-6-yl-acetamide
N-(4-Acetyl-phenyl)-2-[4-(3-hydroxy-phenylamino)-piperidin-1-yl]-acetamide
N-(9-Ethyl-9H-carbazol-3-yl)-2-[4-(4-hydroxy-phenylamino)-piperidin-1-yl]-acetamide
2-[4-(4-Hydroxy-phenylamino)-piperidin-1-yl]-N-(9-oxo-9H-fluoren-3-yl)-acetamide
N-(9-Ethyl-9H-carbazol-3-yl)-2-[4-(2-methoxy-phenylamino)-piperidin-1-yl]-acetamide
2-[4-(2-Methoxy-phenylamino)-piperidin-1-yl]-N-(9-oxo-9H-fluoren-3-yl)-acetamide
2-[4-(2-Methoxy-phenylamino)-piperidin-1-yl]-N-quinolin-6-yl-acetamide
N-(4-Acetyl-phenyl)-2-[4-(2-methoxy-phenylamino)-piperidin-1-yl]-acetamide
2-[4-(2-Methoxy-phenylamino)-piperidin-1-yl]-N-quinolin-3-yl-acetamide
N-(4-Benzoyl-phenyl)-2-[4-(2-methoxy-phenylamino)-piperidin-1-yl]-acetamide
N-(9-Ethyl-9H-carbazol-3-yl)-2-[4-(3-methoxy-phenylamino)-piperidin-1-yl]-acetamide
2-[4-(3-Methoxy-phenylamino)-piperidin-1-yl]-N-(9-oxo-9H-fluoren-3-yl)-acetamide
N-{4-[Ethyl-(2-hydroxy-ethyl)-amino]-phenyl}-2-[4-(3-methoxy-phenylamino)-piperidin-1-yl]-acetamide
N-(4-Benzoyl-phenyl)-2-[4-(3-methoxy-phenylamino)-piperidin-1-yl]-acetamide
N-(9-Ethyl-9H-carbazol-3-yl)-2-[4-(4-methoxy-phenylamino)piperidin-1-yl]-acetamide
2-[4-(4-Methoxy-phenylamino)-piperidin-1-yl]-N-(9-oxo-9H-fluoren-3-yl)-acetamide
N-{4-[Ethyl-(2-hydroxy-ethyl)-amino]-phenyl}-2-[4-(4-methoxy-phenylamino)-piperidin-1-yl]-acetamide
2-[4-(4-Methoxy-phenylamino)-piperidin-1-yl]-N-(4-phenoxy-phenyl)-acetamide
2-[4-(4-Methoxy-phenylamino)-piperidin-1-yl]-N-(5-oxo-5,6,7,8-tetrahydro-naphthalen-2-yl)-acetamide
N-(9-Ethyl-9H-carbazol-3-yl)-2-(4-phenylamino-piperidin-1-yl)-acetamide
N-(9-Oxo-9H-fluoren-3-yl)-2-(4-phenylamino-piperidin-1-yl)-acetamide
N-{4-[Ethyl-(2-hydroxy-ethyl)-amino]-phenyl}-2-(4-phenylamino-piperidin-1-yl)-acetamide
N-(5-Oxo-5,6,7,8-tetrahydro-naphthalen-2-yl)-2-(4-phenylamino-piperidin-1-yl)-acetamide
N-(4-Phenoxy-phenyl)-2-(4-phenylamino-piperidin-1-yl)-acetamide
N-(4-Phenoxy-phenyl)-2-(4-{[(4-phenoxy-phenylcarbamoyl)-methyl]-phenyl-amino}-piperidin-1-yl)-acetamide
2-(4-{(4-Hydroxy-phenyl)-[(9-oxo-9H-fluoren-3-ylcarbamoyl)-methyl]-amino}-piperidin-1-yl)-N-(9-oxo-9H-fluoren-3-yl)-acetamide
optionally in form of a salt, preferably a physiologically acceptable salt, more preferably in form of a physiologically acceptable acid addition salt, most preferably a hydrochloride salt, or a corresponding solvate.

In a further aspect the present invention also provides a process for the preparation of phenylamino-substituted piperidine compounds of general formula (I), wherein R¹- R³, R⁴ to R¹¹, X and Y have the meaning given above, according to which at least one compound of general formula (II), 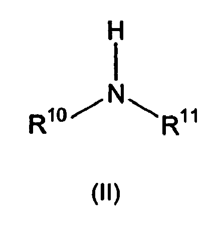 wherein R¹⁰ and R¹¹ have the meaning given above, is reacted with at least one compound of general formula (III), 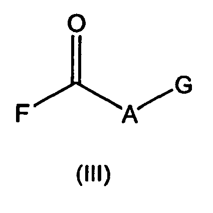 wherein A has the meaning given above, F represents halogen, hydroxy or an O-acyl group and G represents halogen, preferably chlorine, in a suitable reaction medium and preferably in the presence of at least one base selected from the group consisting of diisopropylethylamine, triethylamine, pyridine, dimethylaminopyridine and N-methylmorpholine, to yield a compound of general (IV) 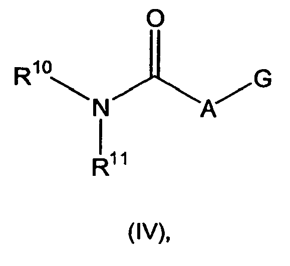 wherein A, G, R¹⁰ and R¹¹ have the above defined meaning, is reacted with at least one piperidine compound of general formula (V) and/or a salt, preferably hydrochloride salt, thereof, 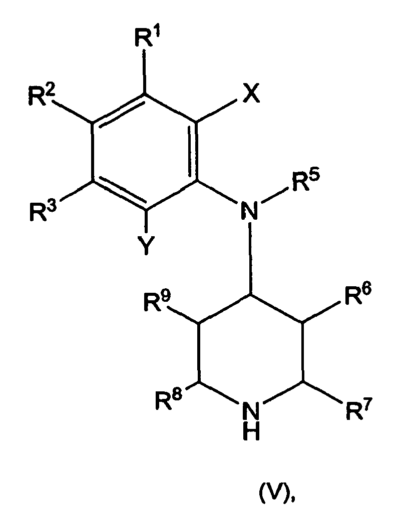 wherein R¹- R³, R⁵ - R⁹, X and Y have the meaning given above, in a suitable reaction medium, optionally in the presence of at least one base and/or at least one auxiliary agent to yield a compound of general formula I.

Compounds of general formula (II), (III) and (IV) are either commercially available or can be produced according to methods known to those skilled in the art.

Preparation of compounds of general formula (V) can be achieved by deprotection reaction from compounds of general formula (VI): 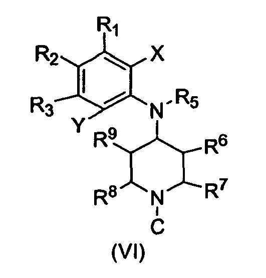 wherein R¹ to R³, R⁵ to R⁹, X and Y have the meaning given above and C is a conventional protecting group like those described in the literature [T.W. Greene and P.G.M. Wuts, Protective groups in organic synthesis, John Wiley & sons, 1999], which can be eliminated by means of methods well known to those skilled in the art.

In another aspect the present invention also provides a process for the preparation of compounds of general formula (VI), according to which at least one compound of general formula (VII), 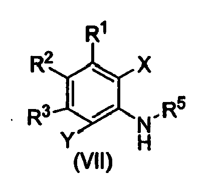 wherein R¹ - R³, R⁵, X and Y have the meaning given above is subjected to a reductive amination with a compound of general formula (VIII): 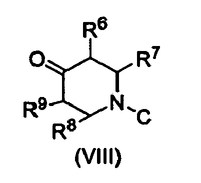 wherein R⁶ to R⁹ and C have the meaning given above. The reductive amination is performed by subjecting a reaction mixture comprising a compound of general formula (VIII), and amino compound of general formula (VII) and a reducing agent in a reaction medium, to microwave radiation for a period of time sufficient to aminate compound (VIII), preferably for 1 to 10 minutes, and at a temperature between 90 to 120°C. The use of microwave irradiation limits the formation of undesirable secondary reaction products, compared to what is obtained in a conventional reductive amination procedure.

This process can be performed as a direct reaction when the carbonyl compound of general formula (VIII) and the amine compound of general formula (VII) are mixed with the reducing agent without prior formation of the intermediate imine or iminium salt. A stepwise or indirect reaction involves the reduction of the preformatted imine in a separate step.

The compounds of general formulas (VII) and (VIII) are either commercially available or can be produced according to methods known to those skilled in the art.

The choice of the reducing agent can be conventionally made by those skilled in the art. Reducing agents useful in this procedure include hydrogen and a catalyst, zinc and HCl, sodium cyanoborohydride, lithium cyanoborohydride, tetrabutylammonium cyanoborohydride, cyanoborohydride on a solid support, sodium cyanoborohydride and dehydrating agents, sodium cyanoborohydride and titanium additives, sodium cyanoborohydride and zinc halide additives, sodium borohydride, sodium borohydride and dehydrating agents, sodium borohydride and titanium additives, sodium borohydride and zinc salt additives, lithium borohydride, potassium borohydride, polymer-supported borohydride, borohydride exchange resin with nickel acetate or palladium acetate, sodium triacetoxyborohydride, sodium triacetoxyborohydride and additives, tetramethylammonium triacetoxyborohydride, sodium cyano-9-borabicyclo[3.3.1]nonane, lithium triethylborohydride, lithium tri(sec-butyl)borohydride, sodium diisopinocampheylcyanoborohydride, amine boranes, borane-pyridine complex and alkylamine boranes. Sodium triacetoxyborohydride is particularly preferred because is non-toxic and generally does not reduce the carbonyl group prior to imine formation.

Suitable reaction media are e.g. organic solvents, such as ethers, preferably diethyl ether, dioxane, tetrahydrofurane, dimethyl glycol ether, or alcohols, e.g. methanol, ethanol, propanol, isopropanol, butanol, isobutanol, tert-butanol, or hydrocarbons, preferably benzene, toluene, xylene, hexane, cyclohexane, petroleum ether, or halogenated hydrocarbons, e.g. dichloromethane, trichloromethane, tetrachloromethane, dichloroethylene, trichloroethylene, chlorobenzene or/and other solvents preferably ethyl acetate, triethylamine, pyridine, dimethulsulfoxide, dimethylformamide, hexamethylphosphoramide, acetonitrile, acetone or nitromethane are included. Mixtures based one or more of the above mentioned solvents may also be used.

According to the invention, the bases that may be used in the process are generally organic or inorganic bases, preferably alkali metal hydroxides, e.g. sodium hydroxide or potassium hydroxide, or obtained from other metals such as barium hidroxyde or different carbonates, preferably potassium carbonate, sodium carbonate, calcium carbonate or alkoxydes, e.g. sodium methoxide potassium methoxide, sodium ethoxide, potassium ethoxide or potassium tert-butoxide, or organic amines, preferably triethylamine, diisopropylethylamine or heterocycles, e.g. 1,4-diazabicyclo[2.2.2]octane, 1,8-diazabicyclo5.4.0]undec-7-ene, pyridine, diamino pydine, dimethylaminopyridine, methylpiperidine or morpholine. Alkaly metals such as sodium or its hydrides, e.g. sodium hydride, may also be used.

The preparation of compounds of general formula (VI) and their use for the preparation of compounds of general formula (I) is illustrated in scheme 1:

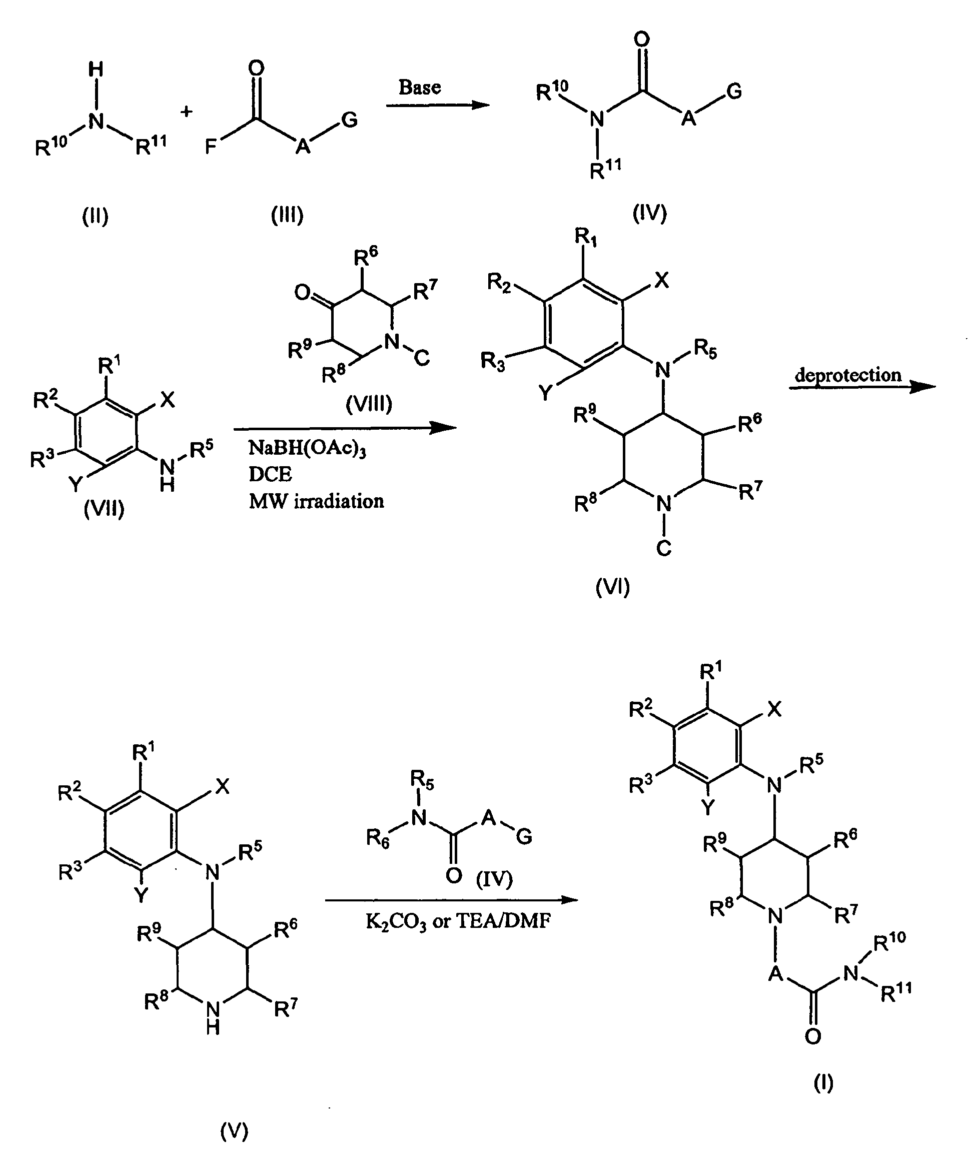

In another aspect, the present invention also provides a process for the preparation of compounds of general formula (I), according to Scheme 2.

The compounds of general formula (IV), wherein R¹⁰, R¹¹, A and G have the meaning given above, were reacted with optionally substituted 4-piperidone hydrochloride of formula (Vlllb) to give the respective piperidones of general formula (IX), wherein A and R⁶ to R¹¹ have the meaning given above.

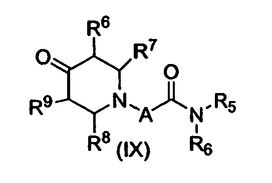

The compounds of formula (IX) can be aminated with compounds of general formula (VII) following a reductive amination process as described above, to yield compounds of general formula (I).

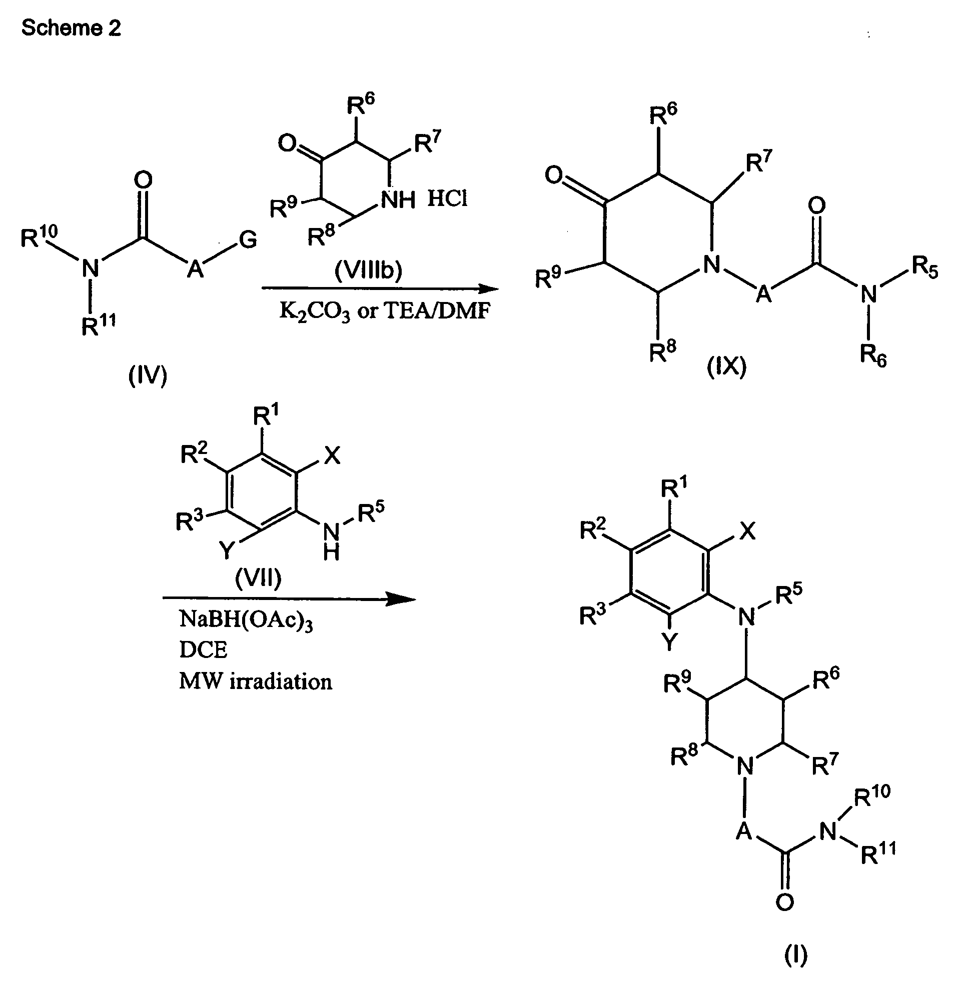

In a further aspect the present invention also provides a process for the preparation of salts of phenylamino-substituted piperidine compounds of general formula (I), wherein at least one compound of general formula (I) is reacted with an inorganic and/or organic acid, preferably in the presence of a suitable reaction medium. Suitable reaction media are the ones given above. Suitable inorganic acid are for example hydrochloric acid, hydrobromic acid, phosphoric acid, sulphuric acid, nitric acid. Suitable organic acids are e.g. citric acid, maleic acid, furmaric acid, tartaric acid or derivatives thereof, such as p-toluenesulfonic acid, methanesulfonic acid or camphersulfonic acid.

In yet a further aspect the present invention also provides a process for the preparation of salts of phenylamino-substituted piperidine compounds of general formula (I), wherein at least one compound of general formula (I) having at least one acidic group is reacted with one or more suitable bases, preferably in the presence of suitable reaction medium. Suitable bases are e.g. hydroxides. Carbonates or alkoxides, which include suitable cations, derived e.g. from alkaline metals, alkaline earth metals or organic cations, e.g. [NHₙR₄₋ₙ]⁺, wherein n is 0, 1, 2, 3 or 4 and R represents a branched or unbranched C₁₋₄ alkyl radical.

Solvates, preferably hydrates, of the phenylamino-substituted piperidine compounds of general formula (I), or corresponding stereoisomers, or corresponding salts may also be obtained by standard procedures known to those skilled in the art.

If the phenylamino-substituted piperidine compounds of general formula (I) are obtained in form of a mixture of stereoisomers, particularly enantiomers or diastereomers, said mixtures may be separated by standard procedures known to those skilled in the art, e.g. chromatographic methods of crystallization with chiral reagents.

The purification and isolation of the phenylamino-substituted piperidine compounds of general formula (I) or a corresponding stereoisomer, or a corresponding salt, or corresponding solvate respectively, if required may be carried out by conventional methods known to those skilled in the art, e.g. chromatographic methods or recrystallization.

The phenylamino-substituted piperidine compounds of general formula (I), their stereoisomers or the respective salts or solvates are toxicologically acceptable and are therefore suitable as pharmaceutical active substances for the preparation of medicaments.

The present invention therefore also provides for a medicament comprising at least one phenylamino-subsbtuted piperidine compound of general formula (I), optionally in form of one of its stereoisomers, preferably enantiomers or diastereomers, its racemate or in form of a mixture of at least two of its stereoisomers, preferably enantiomers or diastereomers, in any mixing ratio, or a physiologically acceptable salt thereof, or a solvate, respectively, and optionally one or more pharmaceutically acceptable adjuvants.

Furthermore, the present invention also provides for a pharmaceutical composition comprising at least one phenylamino-subsfltuted piperidine compound of general formula (I), optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, and optionally one or more pharmaceutically acceptable adjuvants, which is not yet formulated into a medicament.

Preferably the medicament is suitable for the regulation of neuropeptide Y receptors, preferably of neuropeptide Y 5 (NPY5) receptor, regulation of appetite, regulation of body weight, for the regulation of food ingestion (food intake), preferably for the prophylaxis and/or treatment of disorders of food ingestion, preferably obesity, anorexia, bulimia, cachexia or type II diabetes (non insuline dependent diabetes), for the prophylaxis and/or treatment of disorders of the peripheral nervous system, disorders of the central nervous system, diabetes, arthritis, epilepsy, anxiety, depression, cognitive disorders, preferably memory disorders, cardiovascular diseases, pain, hypertensive syndrom, inflammatory diseases or immune diseases.

The present invention also provides for the use of at least one phenylamino-substituted piperidine compound of general formula (I), optionally in form of one of its stereoisomers, preferably enantiomers or diastereomers, its racemate or in form of a mixture of at least two of its stereoisomers, preferably enantiomers or diastereomers, in any mixing ratio, or a physiologically acceptable salt thereof, or a solvate, respectively, for the manufacture of a medicament for the regulation of neuropeptide Y receptors, preferably of neuropeptide Y 5 (NPY5) receptor, regulation of appetite, regulation of body weight, for the regulation of food ingestion (food intake), preferably for the prophylaxis and/or treatment of disorders of food ingestion, preferably obesity, anorexia, bulimia, cachexia or type II diabetes (non insuline dependent diabetes), for the prophylaxis and/or treatment of disorders of the peripheral nervous system, disorders of the central nervous system, diabetes, arthritis, epilepsy, anxiety, depression, cognitive disorders, preferably memory disorders, cardiovascular diseases, pain, hypertensive syndrom, inflammatory diseases or immune diseases.

The medicament may be in any form suitable for the application to humans and/or animals, preferably mammals, and can be produced by standard procedures known to those skilled in the art. The composition of the medicament may vary depending on the route of administration.

The medicament of the present invention may e.g. be administered parentally in combination with conventional injectable liquid carriers, such as water or suitable alcohols. Conventional pharmaceutical adjuvants for injection, such as stabilizing agents, solubilizing agents, and buffers, may be included in such injectable compositions. These medicaments may preferably be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, capsules, lozenges, aqueous or oily solutions, suspensions, emulsions, or dry powdered form suitable for reconstitution with water or other suitable liquid medium before use, for immediate or controlled release.

The liquid oral forms for administration may also contain certain additives such as sweeteners, flavoring, preservatives, and emulsifying agents. Non-aqueous liquid compositions for oral administration may also be formulated, containing e.g. edible oils. Such liquid compositions may be conveniently encapsulated in e.g., gelatin capsules in a unit dosage amount.

The compositions of the present invention may also be administered topically or via a suppository.

The above mentioned compositions include preferably 1 to 60 % by weight of one or more of the phenylamino-substituted piperidine compound of general formula (I), optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, and 40 to 99 % by weight of the appropriate pharmaceutical vehicle(s).

The daily dosage for humans and animals may vary depending on factors that have their basis in the respective species or other factors, such as age, weight or degree of illness and so forth. The daily dosage for mammals including humans usally ranges from 1 milligram to 2000 milligram, preferably 1 to 1500 mg, more preferably 1 to 1000 mg of substace to be administered during one or several intakes.

### Pharmacological Methods:

### Neuropeptide Y₅ Receptor binding studies:

The methods used for membrane preparation and binding are similar to those described by Y. Hu, B. T. Bloomquist et al. in Y. Hu, B. T. Bloomquist et al., The Journal of Biological Chemistry, 1996, 271, 26315-26319 with modifications. Said literature description is herewith incorporated by reference and forms part of the disclosure. Cells C6 were transfected with the rat Y5 receptor. The cells were grown under standard culture conditions in 150 cm² dishes and they were harvested using a rubber scraper and 10 ml PBS. The cells from five dishes were collected and centrifuged 2.500 g for 5 min (4°C). The pellet was washed by resuspending in 3 ml buffer (Tris-HCl 10 mM, pH 7.4), homogenized using a Potter S homogenizer, 10 strokes at 600 rpm and centrifuged 48.000 g for 20 min (4°C). The pellet was resuspended in 8 ml membrane buffer (Tris-HCl 25 mM, NaCl 120 mM, KCl 5 mM, KH₂PO₄ 1,2 mM, CaCl₂ 2,5 mM, MgSO₄ 1,2 mM, BSA 0,15 mg/ml, Bacitracine 0,5 mg/ml, pH 7,4) and rehomogenized using the Potter S, 10 strokes at 600 rpm. The protein concentration in the incubation was 40 µg/ml. The radioligand was [¹²⁵I]-PYY (100 pM) in a total incubation volume of 200 µl. Following incubation at 25°C for 2 h, the reaction was stopped by addition of 5 ml ice-cold buffer (Tris-HCl 25 mM, NaCl 120 mM, KCl 5 mM, KH₂PO4 1,2 mM, CaCl₂ 2,5 mM, MgSO₄ 1,2 mM, pH 7,4) and rapid filtration in a Harvester Brandell Cell using filters (Schleicher & Schuell GF 3362) pretreated for two hours with 0,5% polyethyleneimine. Filters were washed one time with 5 ml ice-cold buffer. The filters were placed into plastic scintilation vials and 5 ml scintilation cocktail Ecoscint H were added. The quantity of radioactivity present was determined in a Wallac Winspectral 1414 counter. Non specific binding was determined in the presence of 1 µM de pNPY. All binding assays were done in triplicate.

### Binding to Neuropeptide Y₂

The experimental protocol follows the method by Y. Dumont et al. as described in Y. Dumont, A. Fournier, S. St-Pierre, R. Quirion: Characterization of Neuropeptide Y Binding Sites in Rat Brain Preparations Using [125I][Leu31, Pro34]Peptide YY and [125I]Peptide YY 3-36 as Selective Y1 and Y2 Radioligands, The Journal of Pharmacology and Experimental Therapeutics, 1995, 272, 673-680, with slight modifications. Said literature description is herewith incorporated by reference and forms part of the disclosure.
Male Wistar rats are sacrificed by decapitation, their brains are rapidly removed and the hypoccampus is dissected. Homogenization is performed in cold conditions in the buffer: 120 mM NaCl, 4.7 mM KCI, 2.2 mM CaCl₂, 1.2 mM KH₂PO₄, 1.2 mM MgSO₄, 25 mM NaHCO₃, 5.5 mM glucose, pH 7.4, by means of a Ultra-Turrax homogenizer for 15 seconds at 13,500 rpm. The ratio between fresh tissue weight and buffer volume is of ten times. The membrane is centrifuged for 10 min at 48,000 g. The supernatant is discarded and the pellet is washed, resuspended and recentrifuged two more times. The final membrane resuspension is performed in the buffer 120 mM NaCl, 4.7 mM KCI, 2.2 mM CaCl₂, 1.2 mM KH₂PO₄, 1.2 mM MgSO₄, 25 mM NaHCO₃,5.5 mM glucose, 0.1% BSA, 0.05% bacitracin, pH 7.4, at a 90 ml/g ratio of fresh issue. The radioligand used is [¹²⁵I]-PYY₃₋₃₆ at the concentration of 28 pM. Incubation volume: 500 µl. Incubation is performed at 25 °C for 150 minutes and ended by rapid filtration in a Harvester Brandel Cell through fiber glass filters of the brand Schleicher & Schuell GF 3362 pretreated with a 0.5% polyethylenimine solution. The filters are cold-washed three times with three milliliters of the same buffer used in homogenization. The filters are transferred to vials and 5 ml of Ecoscint H liquid scintillation cocktail are added to each vial.The vials are allowed to reach steady state for a few hours before counting in a Wallac Winspectral 1414 scintillation counter. Non-specific binding is determined in the presence of 1 µM of pNPY (Neuropeptide Y of porcine origin). The assays are performed in triplicate.

### Behavioural models (Food intake measurements)

In both test, animals rats (Male W, 200-270g, obtained from Harlan, S.A) are used. The rats are acclimatized to the animal facility for at least 5 days before being subjected to any experimental procedure. During this period, animals are housed in groups of five in translucid cages and provided with food and water ad libitum. At least 24 hours before the tests, animals are adapted to single-housing conditions.

### Nocturnal feeding:

Food intake is measured in home cages in order to minimize non-specific stress effects on food intake resulting from changes in housing conditions. Food and water is available ad libitum. Immediately before lights turn off, rats are weighed, randomized and dosed (orally or intraperitoneally), either with vehicle or selected phenylamino-substituted piperidine compounds of general formula (I). Thereafter, rats are returned to home cages and food left on top covers is measured. Remaining food and animal's weight is measured next morning.

The above mentioned methods are described in Ants Kask et al., Europan Journal of Pharmacology 414 (2001) 215-224 and Tumbull et al., Diabetes, Vol. 51, August 2002, which are hereby incorporated by reference and form part of the disclosure.

### Acute effects of selected compounds on food Intake In fasted rats:

Rats are fasted for 23 hours in home cages, and after this period dosed (orally or intraperitoneally), treated either with vehicle or phenylamino-substituted piperidine compound of general formula (I). One hour later preweighed food is left on top covers, and cumulative food intake is measured after 1, 2, 4 and 6 hours.
The methods are described in Ants Kask et al., Europan Journal of Pharmacology 414 (2001) 215-224 and Tumbull et al., Diabetes, Vol. 51, August 2002, , which are hereby incorporated by reference and form part of the disclosure.

The following examples are given to illustrate the present invention, but they do not limit the scope of the present invention.

### Examples:

Prepared according to above-described methods.

### Example A

**2-Chloro-N-(9-oxo-9H-fluoren-3-yl)-acetamide**

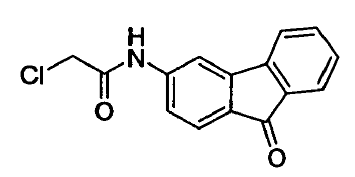

A solution of 3-amino-fluoren-9-one (10mmol), triethylamine (15 mmol), in 25 ml of dried dichloromethane, was cooled to 10°C and a solution of chloroacetyl chloride (10.5 mmol) in 10 ml of dried dichloromethane was slowly added. The reaction was stirred for 12 hour at room temperature. The reaction mixture was washed with 2N HCl (1x30 mL), water (2x30 mL) and dried with sodium sulphate. The solution was concentrated under reduced pressure to give 2-chloro-N-(9-oxo-9H-fluoren-3-yl)-acetamide in 94% yield. This crude material was used without further purification.

### Example B

### 4-(2-Hydroxy phenylamlno)-piperidine-1-carboxylic acid tert-butyl ester

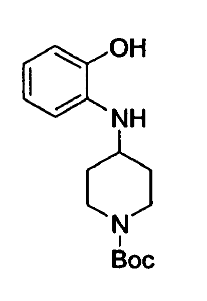

2-Amino-phenol (0.9 mmol) and 4-oxo-piperidine-1-carboxylic acid tert-butyl ester (0.9 mmol) were mixed in 4 ml of 1,2-dichloroethane in a process vial, which was sealed with a septum. Sodium triacetoxyborohydride (2.76 mmol) was added under argon atmosphere. The suspension was subjected to microwave irradiating conditions (CEM Discover^{®} equipped with a CEM Explorer^{®} automated reaction handling module). The reaction mixture was heated for 5 min at 120°C and then cooled. The crude was evaporated to dryness and then suspended in aqueous NaHCO₃. The product was extracted with EtOAc and washed with brine. The EtOAc extract was dried with anhydrous Na₂SO₄, filtered and evaporated to dryness to give the crude product 4-(2-hydroxy-phenylamino)-piperidine-1-carboxylic acid tert-butyl ester. The crude was purified by flash column chromatography (cyclohexane-EtOAc as eluents) by using a CombiFlash Companion^{™} system to yield the title compound (75%).

### Example C

### 2-(Piperidin-4-ylamino)-phenol hydrochloride

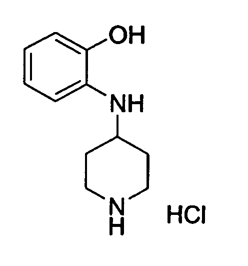

To a stirred solution of 4-(2-hydroxy-phenylamino)-piperidine-1-carboxylic acid tert-butyl ester (2.9 mmol) in EtOAc (12 mL) was added a 5 M solution of hydrogen chloride in diethyl ether (40 mL). The resulting mixture was stirred at room temperature for 4 h. The precipitate formed was collected by filtration, washed with ether and vacuum dried, giving the title compound as a white solid (95%).

### Example D = Example 6

### 2-[4-(2-Hydroxy-phenylamino)-piperidin-1-yl]-N-(9-oxo-9H-fluoren-3-yl)-acetamide

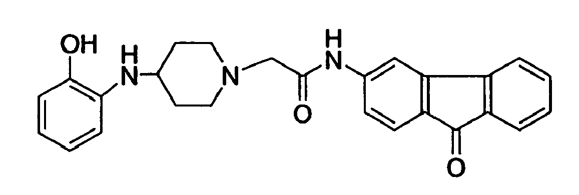

2-Chloro-N-(9-oxo-9H-fluoren-3-yl)-acetamide (0.22 mmol) was added to a suspension of 2-(piperidin-4-ylamino)-phenol hydrochloride (0.2 mmol) in dry DMF (6 mL) at room temperature. The reaction mixture was cooled to 0°C and K₂CO₃ (0.4 mmol) was added. The reaction was allowed to reach room temperature and stirred for 5 hours. Then, the reaction mixture was poured into 30 ml of water containing 4 g of HCl. The aqueous mixture was washed with 4x10 mL of diethyl ether / ethyl acetate 1/1. The combined organic layers were washed with 2x10 mL water and 2x10 mL brine. The organic layers were dried (Na₂SO₄), filtered and solvent was removed in vacuo. The title product was isolated by silica gel chromatography (with dichloromethane-methanol as eluents) as a yellow oil (60%). ¹H-NMR (300 MHz, DMSO-*d*₆) δ ppm 1.51 (q, *J*=10.16 Hz, 2 H) 1.93 (d, *J*=11.28 Hz, 2 H) 2.34 (t, *J*=10.62 Hz, 2 H) 2.85 (d, *J*=11.43 Hz, 2 H) 3.19 (s, 2 H) 4.22 (d, *J*=8.50 Hz, 1 H) 6.38 (t, *J*=7.47 Hz, 1 H) 6.50 - 6.56 (m, 1 H) 6.64 (t, *J*=7.03 Hz, 2 H) 7.37 (t, *J*=7.40Hz, 1 H) 7.54 - 7.71 (m, 5 H) 8.05 (s, 1H) 9.23 (s, 1 H) 10.14 (s, 1 H).

All the other compounds (Examples 1 to 5 and 7 to 38) were done accordingly.

### Examples 1 to 38 :

| **Ex.** | **Structure** | **Name** | **¹H-NMR** | **MS (APCI (M+H)⁺)** |
|---|---|---|---|---|
| 1 | 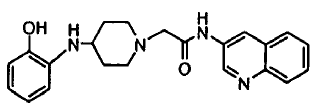 | 2-[4-(2-Hydroxy-phenylamino)-piperidin-1-yl]-N-quinolin-3-yl-acetamide | | 377.10 |
| 2 | 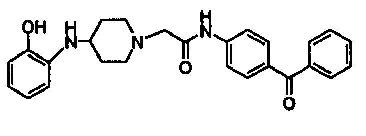 | N-(4-Benzoyl-phenyl)-2-[4-(2-hydroxy-phenylamino)-piperidin-1-yl]-acetamide | | 430.10 |
| 3 | 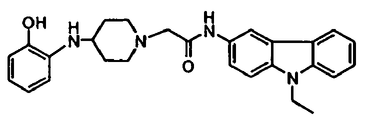 | N-(9-Ethyl-9H-carbazol-3-yl)-2-[4-(2-hydroxy-phenylamino)-piperidin-1-yl]-acetamide | | 443.3 |
| 4 | 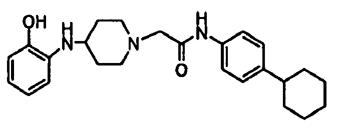 | N-(4-Cyclohexyl-phenyl)-2-[4-(2-hydroxy-phenylamino)-piperidin-1-yl]-acetamide | | 408.3 |
| 5 | 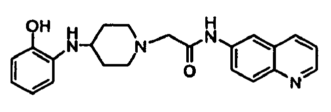 | 2-[4-(2-Hydroxy-phenylamino)-piperidin-1-yl]-N-quinolin-6-yl-acetamide | | 377.18 |
| 6 | 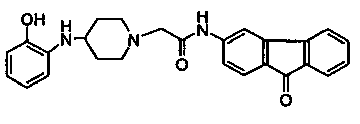 | 2-[4-(2-Hydroxy-phenylamino)-piperidin-1-yl]-N-(9-oxo-9H-fluoren-3-yl)-acetamide | ¹H-NMR (300MHz, DMSO-*d*₆) δ ppm 1.51 (q, *J=*10.16 Hz, 2H) 1.93 (d. *J*=11.28 Hz, 2 H) 2.34 (t, *J*=10.62 Hz, 2 H) 2.85 (d, *J*=11.43Hz, 2 H) 3.19 (s,2 H) 4.22 (d, *J*=8.50 Hz, 1 H) 6.38 (t, *J=*7.47 Hz, 1H) 6.50 - 6.56 (m, 1 H) 6.64 4 (t, *J*=7.03Hz, 2 H) 7.37 (t, *J*=7.40 Hz, 1 H) 7.54 - 7.71 (m, 5 H) 8.05 (s, 1 H) 9.23 (s, 1 H) 10.14 (s, 1 H). | 428.19 |
| 7 | 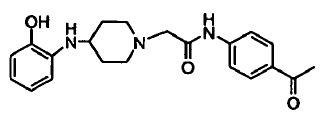 | N-(4-Acetyl-phenyl)-2-[4-(2-hydroxy-phenylamino)-piperidin-1-yl]-acetamide | | 368.19 |
| 8 | 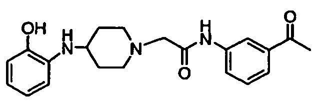 | N-(3-Acetyl-phenyl)-2-[4-(2-hydroxy-phenylamino)-piperidin-1-yl]-acetamide | | 368.1 |
| 9 | 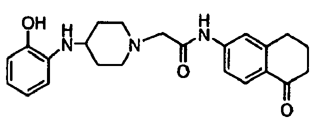 | 2-[4-(2-Hydroxy-phenylamino)-piperidin-1-yl]-N-(5-oxo-5,6,7,8-tetrahydro-naphthalen-2-yl)-acetamide | | 394.1 |
| 10 | 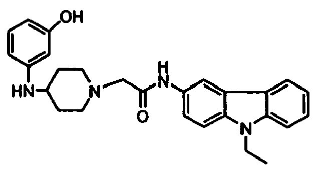 | N-(9-Ethyl-9H-carbazol-3-yl)-2-[4-(3-hydroxy-phenylamino)-piperidin-1-yl]-acetamide | | 443.23 |
| 11 | 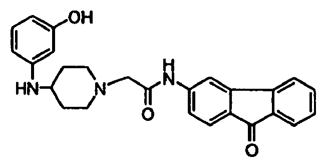 | 2-[4-(3-Hydroxy-phenytamino)-piperidin-1-yl]-N-(9-oxo-9H-fluoren-3-yl)-acetamide | | 428.15 |
| 12 | 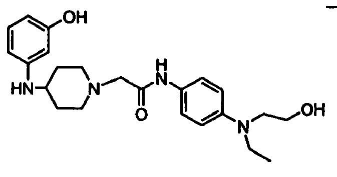 | N-{4-[Ethyl-(2-hydroxy-ethyl)-amino]-phenyl}-2-[4-(3-hydroxy-phenylamino)-piperidin-1-yl]-acetamide | | 413.28 |
| 13 | 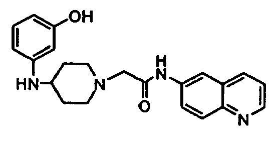 | 2-[4-(3-Hydroxy-phenylamino)-piperldin-1-yl]-N-quinolin-6-yl-acetamide | | 377.19 |
| 14 | 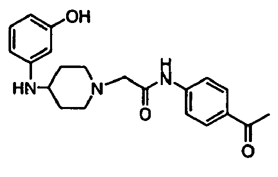 | N-(4-Acetyl-phenyl)-2-[4-(3-hydroxy-phenylamino)-piperidin-1-yl]-acetamide | | 368.19 |
| 15 | 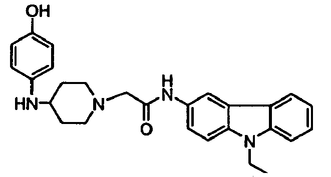 | N-(9-Ethyl-9H-carbazol-3-yl)-2-[4-(4-hydroxy-phenylamino)-piperidin-1-yl]-acetamide | | 443.3 |
| 16 | 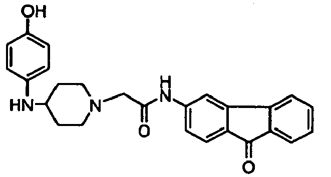 | 2-[4-(4-Hydroxy-phenylamino)-piperidin-1-yl]-N-(9-oxo-9H-fluoren-3-yl)-acetamide | | 428.2 |
| 17 | 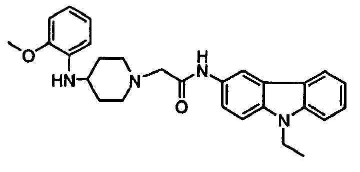 | N-(9-Ethyl-9H- carbazol-3-yl)-2-[4-(2-methoxy-phenylamino)-piperidin-1-yl]-acetamide | | 457.44 |
| 18 | 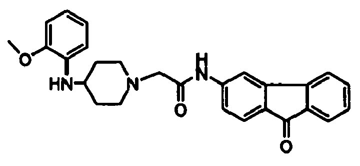 | 2-[4-(2-Methoxy-phonylamino)-pipertdin-1-yl]-N-(9-oxo-9H-fluoren-3-yl)-acetamide | | 442.33 |
| 19 | 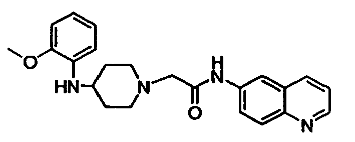 | 2-[4-(2-Methoxy-phenylamino)-piperidin-1-yl]-N-quinolin-6-yl-acetamide | | 391.2 |
| 20 | 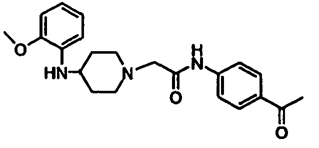 | N-(4-Acetyl-phenyl)-2-[4-(2-methoxy-phenylamino)-piperidin-1-yl]-acetamide | | 382.2 |
| 21 | 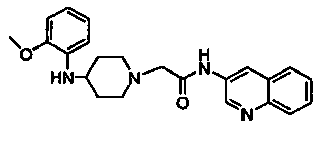 | 2-[4-(2-Methoxy-phenylamino)-pipendin-1-yl]-N-quinolin-3-yl-acetamide | | 391.22 |
| 22 | 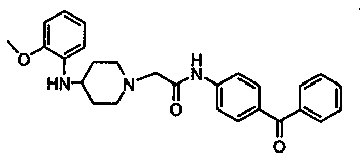 | N-(4-Benzoyl-phenyl)-2-[4-(2-methoxy-phenylamino)-piperidin-1-yl]-acetamide | | 444.5 |
| 23 | 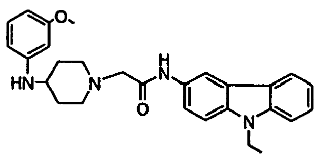 | N-(9-Ethyl-9H-carbazol-3-yl)-2-[4-(3-methoxy-phenylamino)-piperidin-1-yl]-acetamide | | 457.22 |
| 24 | 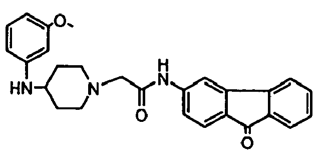 | 2-[4-(3-Methoxy-phenylamino)-piperidin-1-yl]-N-(9-oxo-9H-fluoren-3-yl)-acetamide | | 442.23 |
| 25 | 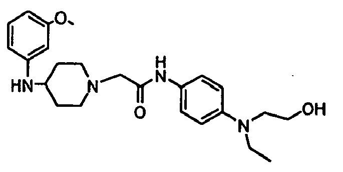 | N-{4-[Ethyl-(2-hydroxy-ethyl)-amino]-phenyl}-2-[4-(3-methoxy-phenylamino)-piperidin-1-yl]-acetamide | | 427.30 |
| 26 | 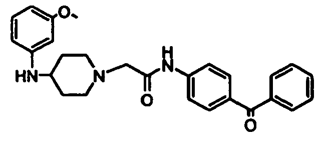 | N-(4-Benzoyl-phenyl)-2-[4-(3-methoxy phenylamino)-piperidin-1-yl]-acetamide | | 444.28 |
| 27 | 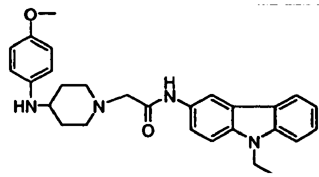 | N-(9-Ethyl-9H-carbazol-3-yl)-2-[4-(4-methoxy-phenylamino)-piperidin-1-yl]-acetamide | | 457.09 |
| 28 | 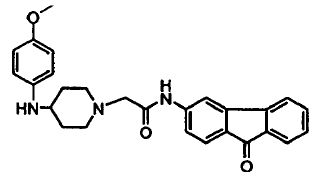 | 2-[4-(4-Methoxy-phenylamino)-piperidin-1-yl]-N-(9-oxo-9H-fluoren-3-yl)-acetamide | | 442.2 |
| 29 | 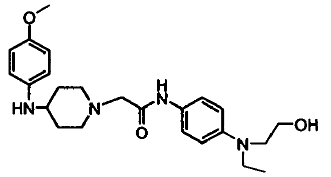 | N-{4-[Ethyl-(2-hydroxy-ethyl)-amino]-phenyl}-2-[4-(4-methoxy-phenytamino)-piperidin-1-yl]-acetamide | | 427.37 |
| 30 | 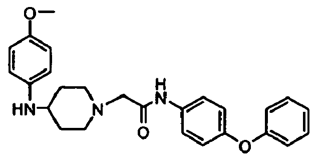 | 2-[4-(4-Methoxy-phenylamino)-piperidin-1-yl]-N-(4-phenoxy-phenyl)-acetamide | | 432.03 |
| 31 | 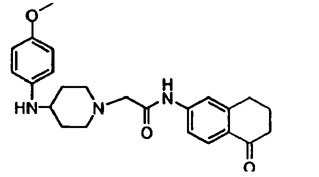 | 2-[4-(4-Methoxy-phenylamino)-piperidin-1-yl]-N-(5-oxo-5,6,7,8-tetrahydro-naphthalen-2-yl)-acetamide | | 408.11 |
| 32 | 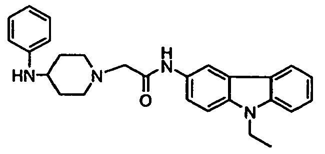 | N-(9-Ethyl-9H-carbazol-3-yl)-2-(4-phenylamino-piperidin-1-yl)-acetamide | | 427.22 |
| 33 | 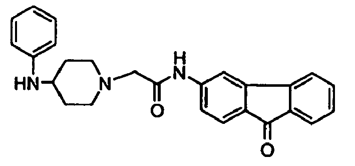 | N-(9-Oxo-9H-fluoren-3-yl)-2-(4-phenylamino-piperidin-1-yl)-acetamide | | 412.3 |
| 34 | 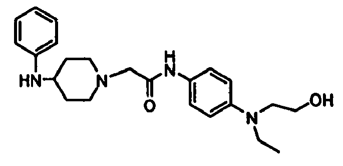 | N-{4-[Ethyl-(2-hydroxy-ethyl)-amino]-phenyl}-2-(4-phenylamino-piperidin-1-yl)-acetamide | | 397.4 |
| 35 | 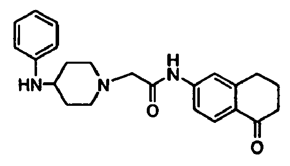 | N-(5-Oxo-5,6,7,8-tetrahydro-naphthalen-2-yl)-2-(4-phenylamino-piperidin-1-yl)-acetamide | | 378.23 |
| 36 | 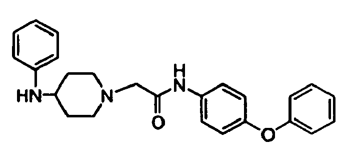 | N-(4-Phenoxy-phenyl)-2-(4-phenylamino-piperidin-1-yl)-acetamide | | 402.4 |
| 37 | 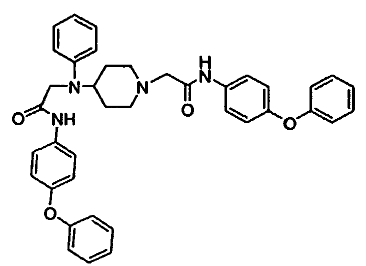 | N-(4-Phenoxy-phenyl)-2-(4-{[(4-phenoxy-phenylcarbamoyl)-methyl]-phenyl-amino}-piperidin-1-yl)-acetamide | | 627.04 |
| 38 | 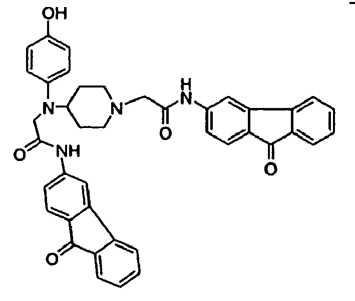 | 2-(4-{(4-Hydroxy-phenyl)-[(9-oxo-9H-fluoren-3-ylcarbamoyl)-methyl]-amino}-piperidin-1-yl)-N-(9-oxo-9H-fluoren-3-yl)-acetamide | | 663.2 |

### Formulation Example

### Example of formula per tablet:

| | |
|---|---|
| Compound according to example 32 | 5 mg |
| Lactose | 60 mg |
| Crystalline cellulose | 25 mg |
| Povidone K 90 | 5 mg |
| Pregelanitized starch | 3 mg |
| Colloidal silica dioxide | 1 mg |
| Magnesium stearate | 1 mg |
| Total weight per tablet | 100 mg |

The above mentioned ingredients were mixed and compressed into a tablet by conventional methods known to those skilled in the art.

### Pharmacological Data:

### (a)

According to the methods given above Neuropeptide Y₅-Binding of the phenylamino-substituted piperidine compounds of general formula (I) has been determined. Some of the lC₅₀-values values are given in the following table 1.

**Table 1:**

| **Compound according to Example** | **Neuropeptide Y₅ Binding IC₅₀ (nM)** | **Neuropeptide Y₅ Binding % Inhibition (10⁻⁶M)** |
|---|---|---|
| 1 | | 5.6 |
| 2 | | 62.5 |
| 3 | 66.1 | 97.8 |
| 4 | | 35.3 |
| 5 | | 22.7 |
| 6 | 57.5 | 57.5 |
| 7 | | 14 |
| 9 | | 44.7 |
| 10 | | 104.9 |
| 11 | | 76.9 |
| 12 | | 51.2 |
| 13 | | 27.8 |
| 15 | | 74.6 |
| 16 | | 100.7 |
| 17 | | 101.3 |
| 18 | | 96.1 |
| 21 | | 19.6 |
| 22 | | 55.7 |
| 23 | | 96.9 |
| 24 | | 97.5 |
| 25 | | 51.6 |
| 26 | | 37 |
| 27 | | 91.1 |
| 28 | | 97.3 |
| 29 | | 69 |
| 32 | 23.7 | 80.7 |
| 33 | | 108.5 |
| 34 | | 72.6 |
| 35 | | 67.1 |
| 38 | | 45.1 |

## Claims

1. Phenylamino-substituted piperidine compound of general formula (I) 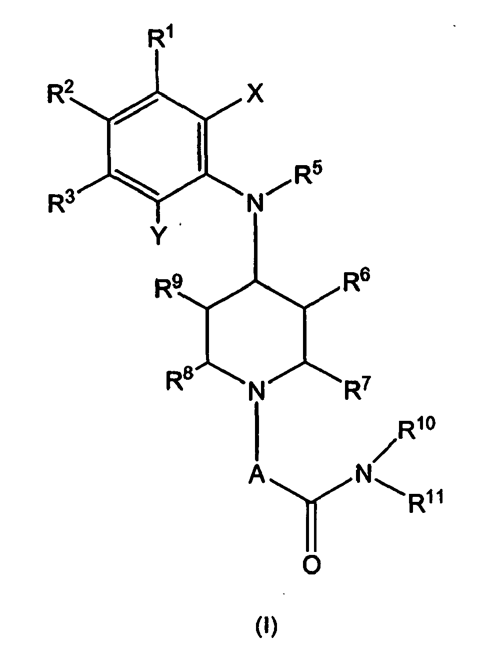 wherein
X and Y are each independently selected from the group consisting of hydrogen, halogen, a nitro, -OR¹², -OC(=O)R¹³, -SR¹⁴, -SOR¹⁴, -SO₂R¹⁴, -NH-SO₂R¹⁴, -SO₂NH₂ and -NR¹⁵R¹⁶ moiety;
R¹, R², R³, are each independently selected from the group consisting of hydrogen; halogen; an unbranched or branched, saturated or unsaturated, optionally at least mono-substituted aliphatic radical; a nitro, cyano, -OR¹², -OC(=O)R¹³, -SR¹⁴, -SOR¹⁴, -SO₂R¹⁴, -NH-SO₂R¹⁴, -SO₂NH₂ and -NR¹⁵R¹⁶ moiety,
R⁵ represents hydrogen; an unbranched or branched, saturated or unsaturated, optionally at least mono-substituted aliphatic radical; or a group 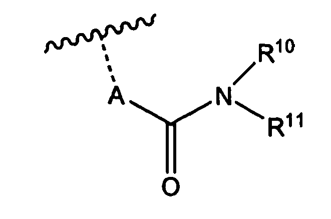 R⁶, R⁷, R⁸, R⁹ are each independently selected from the group consisting of hydrogen; an unbranched or branched, saturated or unsaturated, optionally at least mono-substituted aliphatic radical; a cyano or a COOR¹⁷ moiety,
A represents a bridge member -CHR¹⁸- or -CHR¹⁸-CH₂-;
R¹⁰ represents hydrogen; an unbranched or branched, saturated or unsaturated, optionally at least mono-substituted aliphatic radical;
R¹¹ represents an unbranched or branched, saturated or unsaturated, optionally at least mono-substituted aliphatic radical; a saturated or unsaturated, optionally at least mono-substituted cycloaliphatic radical, which may be bonded via an optionally at least mono-substituted alkylene group and/or may be condensed with an optionally at least mono-substituted mono- or polycyclic ringsystem, and which optionally may be bonded to another mono-or polycyclic ringsystem through either a direct bond, an optionally at least mono-substituted alkylene group, an ether bond or a keto-group; or an optionally at least mono substituted aryl radical, which may be bonded via an optionally at least mono-substituted alkylene group and/or may be condensed with an optionally at least mono-substituted mono- or polycyclic ringsystem, which optionally may be bonded to another mono-or polycyclic ringsystem through either a direct bond, an optionally at least mono-substituted alkylene group, an ether bond or a keto-group; or an optionally at least mono substituted heterocyclyl radical, which may be bonded via an optionally at least mono-substituted alkylene group and/or may be condensed with an optionally at least mono-substituted mono- or polycyclic ringsystem, and which optionally may be bonded to another mono-or polycyclic ringsystem through either a direct bond, an optionally at least mono-substituted alkylene group, an ether bond or a keto-group; or
R¹⁰ and R¹¹ together with the bridging nitrogen atom form an optionally at least mono-substituted, saturated, unsaturated or aromatic heterocyclic ring that may contain at least one further heteroatom as a ring member and/or may be condensed with an optionally at least mono-substituted mono- or polycyclic ringsystem;
R¹², R¹³, R¹⁴ are hydrogen; or an unbranched or branched, saturated or unsaturated, optionally at least mono-substituted aliphatic radical;
R¹⁵ and R¹⁶ each are independently selected from the group consisting of hydrogen; or an unbranched or branched, saturated or unsaturated, optionally at least mono-substituted aliphatic radical;
R¹⁷ represents hydrogen; or an unbranched or branched, saturated or unsaturated, -optionally at least mono-substituted aliphatic radical;
R¹⁸ represents hydrogen; or an unbranched or branched, saturated or unsaturated, optionally at least mono-substituted aliphatic radical;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

2. Compounds according to claim 1, **characterized in that** X and Y are each independently selected from the group consisting of hydrogen, halogen, a nitro, or - OR¹² moiety; preferably X and Y are each independently selected from the group consisting of hydrogen, or -OR¹² moiety.

3. Compounds according to claim 1, **characterized in that** R¹, R², R³, are each independently selected from the group consisting of hydrogen; halogen; an unbranched or branched, saturated or unsaturated, optionally at least mono-substituted aliphatic radical; a nitro, -OR¹², -OC(=O)R¹³, and -NR¹⁵R¹⁶ moiety; preferably R¹, R², R³, are each independently selected from the group consisting of hydrogen; or -OR¹² moiety;

4. Compounds according to any one of claims 1 to 3, **characterized in that** R¹² , R¹³ , R¹⁴ are hydrogen; or an optionally substituted branched or unbranched C₁₋₄-alkyl radical; preferably R¹² , R¹³, R¹⁴ represent H, CH₃, CF₃, CH₂CH₃, CH₂CH₂CH₃, CH(CH₃)₂ or C(CH₃)₃.

5. Compounds according to any one of claims 1 to 3, **characterized in that** R¹⁵ and R¹⁶ are each independently selected from the group consisting of H, or a C₁₋₄-alkyl radical, preferably selected from the group consisting of H, CH₃, and C₂H₅.

6. Compounds according to any one of claims 1 to 5, **characterized in that** R⁵ represents H or a branched or unbranched C₁₋₃-alkyl radical, or a group 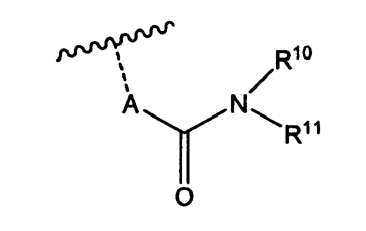 peferably R⁵ represents H, CH₃ or CH₂CH₃; or a group 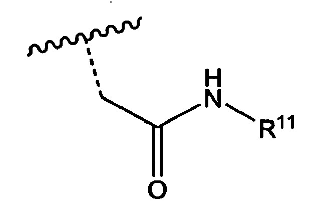 most preferably R⁵ represents H; or a group

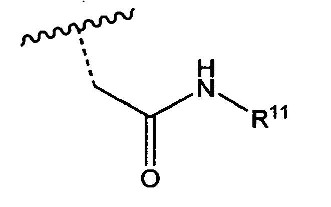

7. Compounds according to any one of claims 1 to 6, **characterized in that** R⁶, R⁷, R⁸, R⁹ are each independently selected from the group consisting of H, a branched or unbranched C₁₋₃-alkyl radical, or a cyano-moiety; preferably from the group consisting of H, CH₃, CH₂CH₃ and a cyano-moiety, more preferably all represent H.

8. Compounds according to any one of claims 1 to 6, **characterized in that** R¹⁷ represents H, or a C₁₋₄-alkyl radical; preferably R¹⁷ represents H, CH₃ or C₂H₅.

9. Compounds according to any one of claims 1 to 8, **characterized in that** R¹⁸ represents H, or a C₁₋₄-alkyl radical or a phenyl radical, preferably R¹⁸ represents H, or CH₃, more preferably R¹⁸ represents H.

10. Compounds according to any one of claims 1 to 9, **characterized in that** R¹⁰ represents hydrogen or a branched or unbranched C₁₋₄-alkyl radical; preferably R¹⁰ represents H, CH₃, CH₂CH₃, CH₂CH₂CH₃, CH(CH₃)₂ or C(CH₃)₃; more preferably R¹⁰ represents H.

11. Compounds according to any one of claims 1 to 10, **characterized in that** R¹¹ is selected from the group consisting of an optionally at least mono substituted aryl radical, which may be bonded via an optionally at least mono-substituted alkylene group and/or may be condensed with an optionally at least mono-substituted mono-or polycyclic ringsystem, which optionally may be bonded to another mono-or polycyclic ringsystem through either a direct bond, an optionally at least mono-substituted alkylene group, an ether bond or a keto-group; or an optionally at least mono substituted heterocyclyl radical, which may be bonded via an optionally at least mono-substituted alkylene group and/or may be condensed with an optionally at least mono-substituted mono- or polycyclic ringsystem, and which optionally may be bonded to another mono-or polycyclic ringsystem through either a direct bond, an optionally at least mono-substituted alkylene group, an ether bond or a keto-group;
preferably R¹¹ is selected from the group consisting of an optionally at least mono substituted phenyl or pyridine radical, which may be condensed with an optionally at least mono-substituted mono- or polycyclic ringsystem, and which optionally may be bonded to another mono-or polycyclic ringsystem through either a direct bond, an optionally at least mono-substituted alkylene group, an ether bond or a keto-group;
more preferably R¹¹ is selected from the group consisting of 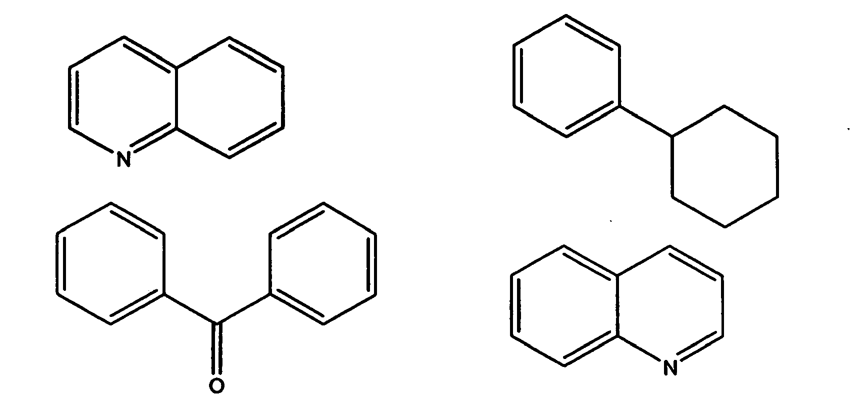 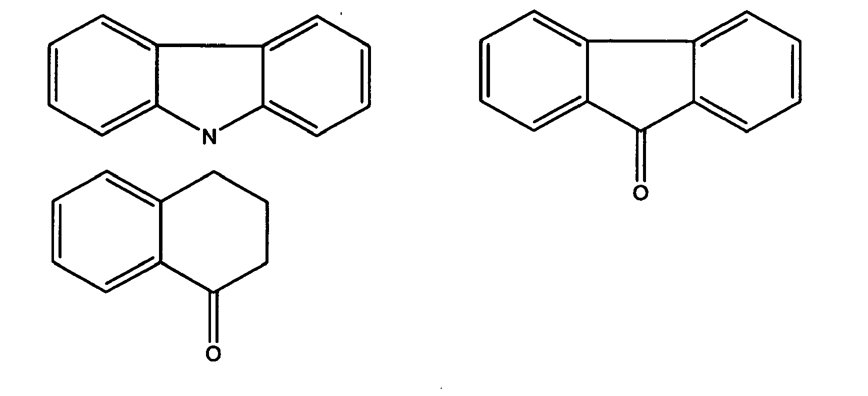 optionally at least monosubstituted.

12. Compounds according to any one of claims 1 to 9, **characterized in that** R¹⁰ and R¹¹ together with the bridging nitrogen atom form a saturated, 6-membered heterocyclic ring, which is at least mono-substituted with a methyl radical and/or condensed with an unsubstituted or at least mono-substituted phenyl- or cyclohexyl-radical, said phenyl- or cyclohexyl-radical preferably being at least mono-substituted with F and/or OCH₃.

13. Compound according to claim 1 of general formula (Ia) 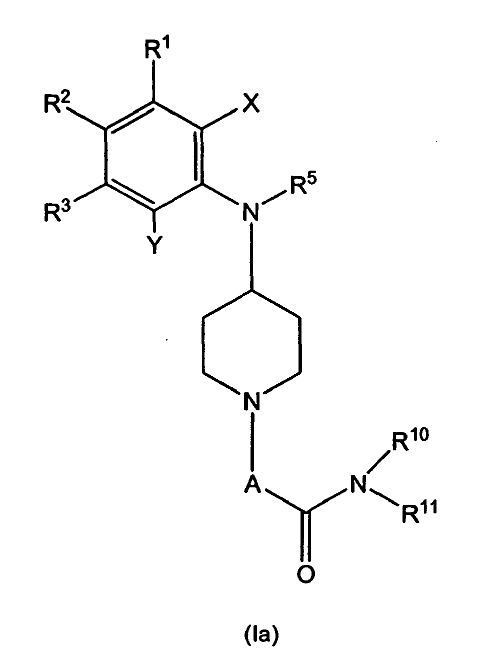 wherein
X and Y are each independently selected from the group consisting of hydrogen, halogen, a nitro, or -OR¹² moiety;
R¹, R², R³, are each independently selected from the group consisting of hydrogen; halogen; an unbranched or branched, saturated or unsaturated, optionally at least mono-substituted aliphatic radical; a nitro, -OR¹², -OC(=O)R¹³, and -NR¹⁵R¹⁶ moiety,
R⁵ represents hydrogen; an unbranched or branched, saturated or unsaturated, optionally at least mono-substituted aliphatic radical; or a group 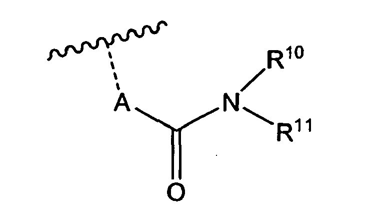 A represents a bridge member -CH₂- or -CH₂-CH₂-
R¹⁰ represents hydrogen; an unbranched or branched, saturated or unsaturated, optionally at least mono-substituted aliphatic radical;
R¹¹ represents an unbranched or branched, saturated or unsaturated, optionally at least mono-substituted aliphatic radical; a saturated or unsaturated, optionally at least mono-substituted cycloaliphatic radical, which may be bonded via an optionally at least mono-substituted alkylene group and/or may be condensed with an optionally at least mono-substituted mono- or polycyclic ringsystem, and which optionally may be bonded to another mono-or polycyclic ringsystem through either a direct bond, an optionally at least mono-substituted alkylene group, an ether bond or a keto-group; or an optionally at least mono substituted aryl radical, which may be bonded via an optionally at least mono-substituted alkylene group and/or may be condensed with an optionally at least mono-substituted mono- or polycyclic ringsystem, which optionally may be bonded to another mono-or polycyclic ringsystem through either a direct bond, an optionally at least mono-substituted alkylene group, an ether bond or a keto-group; or an optionally at least mono substituted heterocyclyl radical, which may be bonded via an optionally at least mono-substituted alkylene group and/or may be condensed with an optionally at least mono-substituted mono- or polycyclic ringsystem, and which optionally may be bonded to another mono-or polycyclic ringsystem through either a direct bond, an optionally at least mono-substituted alkylene group, an ether bond or a keto-group; or
R¹⁰ and R¹¹ together with the bridging nitrogen atom form an optionally at least mono-substituted, saturated, unsaturated or aromatic heterocyclic ring that may contain at least one further heteroatom as a ring member and/or may be condensed with an optionally at least mono-substituted mono- or polycyclic ringsystem;
R¹², R¹³ are hydrogen; or an unbranched or branched, saturated or unsaturated, optionally at least mono-substituted aliphatic radical;
R¹⁵ and R¹⁶ each are independently selected from the group consisting of hydrogen; or an unbranched or branched, saturated or unsaturated, optionally at least mono-substituted aliphatic radical;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

14. Compounds according to claim 13, **characterized in that** X and Y are each independently selected from the group consisting of hydrogen, or -OR¹² moiety.

15. Compounds according to claim 13 or 14, **characterized in that** R¹, R², R³, are each independently selected from the group consisting of hydrogen; F, Cl, Br; an optionally substituted branched or unbranched C₁₋₄-alkyl radical; an -OR¹² moiety; preferably R¹, R², R³, are each independently selected from the group consisting of hydrogen; or - OR¹² moiety;

16. Compounds according to any one of claims 13 to 15, **characterized in that** R¹², R¹³ are hydrogen; or an optionally substituted branched or unbranched C₁₋₄-alkyl radical; preferably R¹², R¹³ represent H, CH₃, CF₃, CH₂CH₃, CH₂CH₂CH₃, CH(CH₃)₂ or C(CH₃)₃.

17. Compounds according to claim 13, **characterized in that** R¹⁵ and R¹⁶ are each independently selected from the group consisting of H, or a C₁₋₄-alkyl radical, preferably selected from the group consisting of H, CH₃, and C₂H₅.

18. Compounds according to any one of claims 1 to 5, **characterized in that** R⁵ represents H or a branched or unbranched C₁₋₃-alkyl radical, or a group 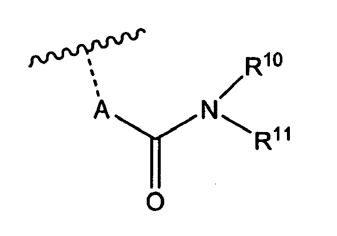 peferably R⁵ represents H, CH₃ or CH₂CH₃; or a group 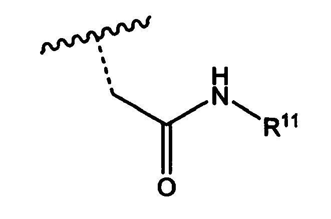 most preferably R⁵ represents H; or a group

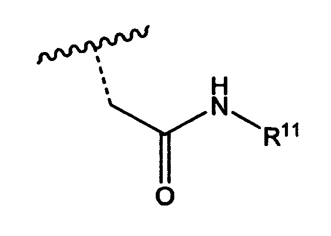

19. Compounds according to any one of claims 13 to 18, **characterized in that** R¹⁰ represents hydrogen or a branched or unbranched C₁₋₄-alkyl radical; preferably R¹⁰ represents H, CH₃, CH₂CH₃, CH₂CH₂CH₃, CH(CH₃)₂ or C(CH₃)₃; more preferably R¹⁰ represents H.

20. Compounds according to any one of claims 13 to 19, **characterized in that** R¹¹ is selected from the group consisting of an optionally at least mono substituted aryl radical, which may be bonded via an optionally at least mono-substituted alkylene group and/or may be condensed with an optionally at least mono-substituted mono-or polycyclic ringsystem, which optionally may be bonded to another mono-or polycyclic ringsystem through either a direct bond, an optionally at least mono-substituted alkylene group, an ether bond or a keto-group; or an optionally at least mono substituted heterocyclyl radical, which may be bonded via an optionally at least mono-substituted alkylene group and/or may be condensed with an optionally at least mono-substituted mono- or polycyclic ringsystem, and which optionally may be bonded to another mono-or polycyclic ringsystem through either a direct bond, an optionally at least mono-substituted alkylene group, an ether bond or a keto-group;
preferably R¹¹ is selected from the group consisting of an optionally at least mono substituted phenyl or pyridine radical, which may be condensed with an optionally at least mono-substituted mono- or polycyclic ringsystem, and which optionally may be bonded to another mono-or polycyclic ringsystem through either a direct bond, an optionally at least mono-substituted alkylene group, an ether bond or a keto-group; more preferably R¹¹ is selected from the group consisting of 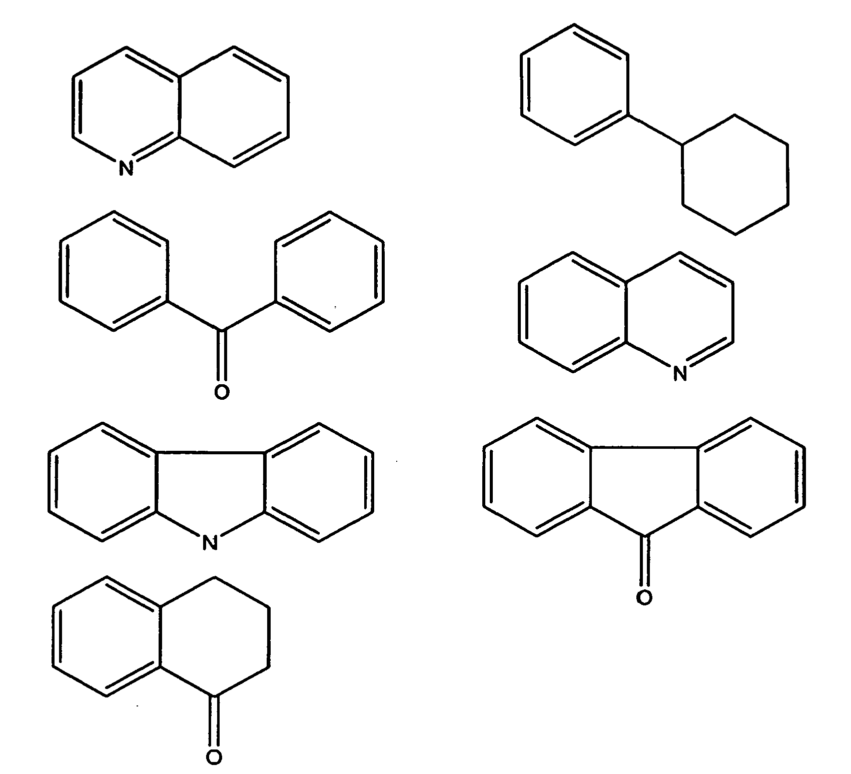 optionally at least monosubstituted.

21. Compound according to claim 1 of general formula (Ib) 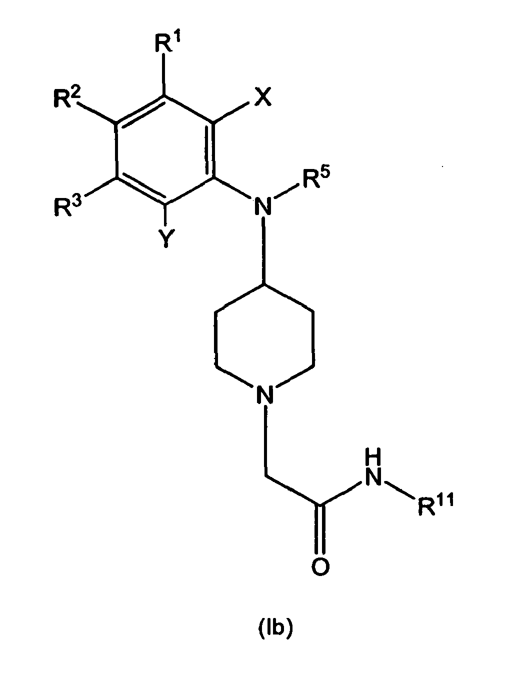 wherein
X and Y are each independently selected from the group consisting of hydrogen, or - OR¹² moiety;
R¹, R², R³, are each independently selected from the group consisting of hydrogen; or -OR¹² moiety,
R⁵ represents hydrogen; or a group 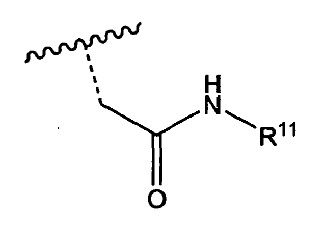 R¹¹ represents an unbranched or branched, saturated or unsaturated, optionally at least mono-substituted aliphatic radical; a saturated or unsaturated, optionally at least mono-substituted cycloaliphatic radical, which may be bonded via an optionally at least mono-substituted alkylene group and/or may be condensed with an optionally at least mono-substituted mono- or polycyclic ringsystem, and which optionally may be bonded to another mono-or polycyclic ringsystem through either a direct bond, an optionally at least mono-substituted alkylene group, an ether bond or a keto-group; or an optionally at least mono substituted aryl radical, which may be bonded via an optionally at least mono-substituted alkylene group and/or may be condensed with an optionally at least mono-substituted mono- or polycyclic ringsystem, which optionally may be bonded to another mono-or polycyclic ringsystem through either a direct bond, an optionally at least mono-substituted alkylene group, an ether bond or a keto-group; or an optionally at least mono substituted heterocyclyl radical, which may be bonded via an optionally at least mono-substituted alkylene group and/or may be condensed with an optionally at least mono-substituted mono- or polycyclic ringsystem, and which optionally may be bonded to another mono-or polycyclic ringsystem through either a direct bond, an optionally at least mono-substituted alkylene group, an ether bond or a keto-group;
R¹² is hydrogen or CH₃, C₂H₅ or CF₃;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

22. Compounds according to any one of claims 21 to, **characterized in that** R¹, R², R³, X or Y are each independently selected from the group consisting of H, OH or OCH₃.

23. Compounds according to any one of claims 13 to 15, **characterized in that** R¹², R¹³ are hydrogen; or an optionally substituted branched or unbranched C₁₋₄-alkyl radical; preferably R¹², R¹³ represent H, CH₃, CF₃, CH₂CH₃, CH₂CH₂CH₃, CH(CH₃)₂ or C(CH₃)₃.

24. Compounds according to any one of claims 21 to 23, **characterized in that** R⁵ represents H.

25. Compounds according to any one of claims 21 to 23, **characterized in that** R⁵ represents

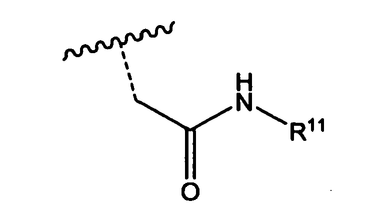

26. Compounds according to any one of claims 20 to 25, **characterized in that** R¹¹ is selected from the group consisting of an optionally at least mono substituted aryl radical, which may be bonded via an optionally at least mono-substituted alkylene group and/or may be condensed with an optionally at least mono-substituted mono-or polycyclic ringsystem, which optionally may be bonded to another mono-or polycyclic ringsystem through either a direct bond, an optionally at least mono-substituted alkylene group, an ether bond or a keto-group; or an optionally at least mono substituted heterocyclyl radical, which may be bonded via an optionally at least mono-substituted alkylene group and/or may be condensed with an optionally at least mono-substituted mono- or polycyclic ringsystem, and which optionally may be bonded to another mono-or polycyclic ringsystem through either a direct bond, an optionally at least mono-substituted alkylene group, an ether bond or a keto-group;
preferably R¹¹ is selected from the group consisting of an optionally at least mono substituted phenyl or pyridine radical, which may be condensed with an optionally at least mono-substituted mono- or polycyclic ringsystem, and which optionally may be bonded to another mono-or polycyclic ringsystem through either a direct bond, an optionally at least mono-substituted alkylene group, an ether bond or a keto-group;
more preferably R¹¹ is selected from the group consisting of 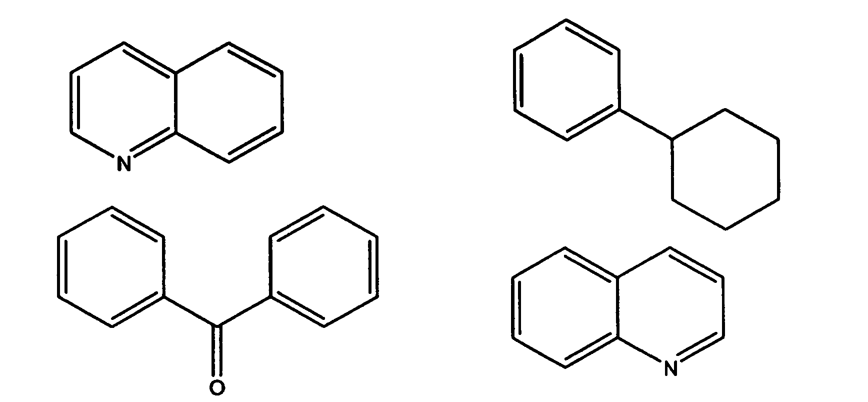 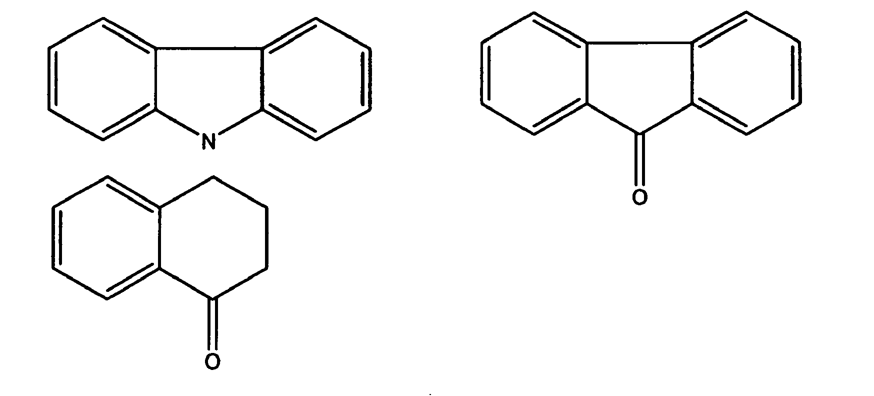 optionally at least monosubstituted.

27. Compounds according to one or more of claims 1 to 26:
2-[4-(2-Hydroxy-phenylamino)-piperidin-1-yl]-N-quinolin-3-yl-acetamide
N-(4-Benzoyl-phenyl)-2-[4-(2-hydroxy-phenylamino)-piperidin-1-yl]-acetamide
N-(9-Ethyl-9H-carbazol-3-yl)-2-[4-(2-hydroxy-phenylamino)-piperidin-1-yl]-acetamide
N-(4-Cyclohexyl-phenyl)-2-[4-(2-hydroxy-phenytamino)-piperidin-1-yl]-acetamide
2-[4-(2-Hydroxy-phenylamino)-piperidin-1-yl]-N-quinolin-6-yl-acetamide
2-[4-(2-Hydroxy-phenylamino)-piperidin-1-yl]-N-(9-oxo-9H-fluoren-3-yl)-acetamide
N-(4-Acetyl-phenyl)-2-[4-(2-hydroxy-phenylamino)-piperidin-1-yl]-acetamide
N-(3-Acetyl-phenyl)-2-[4-(2-hydroxy-phenylamino)-piperidin-1-yl]-acetamide
2-[4-(2-Hydroxy-phenylamino)-piperidin-1-yl]-N-(5-oxo-5,6,7,8-tetrahydro-naphthalen-2-yl)-acetamide
N-(9-Ethyl-9H-carbazol-3-yl)-2-[4-(3-hydroxy-phenylamino)-piperidin-1-yl]-acetamide
2-[4-(3-Hydroxy-phenylamino)-piperidin-1-yl]-N-(9-oxo-9H-fluoren-3-yl)-acetamide
N-{4-[Ethyl-(2-hydroxy-ethyl)amino]-phenyl}-2-[4-(3-hydroxy-phenylamino)-piperidin-1-yl]-acetamide
2-[4-(3-Hydroxy-phenylamino)-piperidin-1-yl]-N-quinolin-6-yl-acetamide
N-(4-Acetyl-phenyl)-2-[4-(3-hydroxy-phenylamino)piperidin-1-yl]-acetamide
N-(9-Ethyl-9H-carbazol-3-yl)-2-[4-(4-hydroxy-phenylamino)-piperidin-1-yl]-acetamide
2-[4-(4-Hydroxy-phenylamino)-piperidin-1-yl]-N-(9-oxo-9H-fluoren-3-yl)-acetamide
N-(9-Ethyl-9H-carbazol-3-yl)-2-[4-(2-methoxy-phenylamino)-piperidin-1-yl]-acetamide
2-[4-(2-Methoxy-phenylamino)-piperidin-1-yl]-N-(9-oxo-9H-fluoren-3-yl)-acetamide
2-[4-(2-Methoxy-phenylamino)-piperidin-1-yl]-N-quinolin-6-yl-acetamide
N-(4-Acetyl-phenyl)-2-[4-(2-methoxy-phenylamino)-piperidin-1-yl]-acetamide
2-[4-(2-Methoxy-phenylamino)-piperidin-1-yl]-N-quinolin-3-yl-acetamide
N-(4-Benzoyl-phenyl)-2-[4-(2-methoxy-phenylamino)-piperidin-1-yl]-acetamide
N-(9-Ethyl-9H-carbazol-3-yl)-2-[4-(3-methoxy-phenylamino)-piperidin-1-yl]-acetamide
2-[4-(3-Methoxy-phenylamino)-piperidin-1-yl]-N-(9-oxo-9H-fluoren-3-yl)-acetamide
N-{4-[Ethyl-(2-hydroxy-ethyl)-amino]-phenyl}-2-[4-(3-methoxy-phenylamino)-piperidin-1-yl]-acetamide
N-(4-Benzoyl-phenyl)-2-[4-(3-methoxy-phenylamino)-piperidin-1-yl]-acetamide
N-(9-Ethyl-9H-carbazol-3-yl)-2-[4-(4-methoxy-phenylamino)-piperidin-1-yl]-acetamide
2-[4-(4-Methoxy-phenylamino)-piperidin-1-yl]-N-(9-oxo-9H-fluoren-3-yl)-acetamide
N-{4-[Ethyl-(2-hydroxy-ethyl)-amino]-phenyl}-2-[4-(4-methoxy-phenylamino)-piperidin-1-yl]-acetamide
2-[4-(4-Methoxy-phenylamino)-piperidin-1-yl]-N-(4-phenoxy-phenyl)-acetamide
2-[4-(4-Methoxy-phenylamino)-piperidin-1-yl]-N-(5-oxo-5,6,7,8-tetrahydro-naphthalen-2-yl)-acetamide
N-(9-Ethyl-9H-carbazol-3-yl)-2-(4-phenylamino-piperidin-1-yl)-acetamide
N-(9-Oxo-9H-fluoren-3-yl)-2-(4-phenylamino-piperidin-1-yl)-acetamide
N-{4-[Ethyl-(2-hydroxy-ethyl)-amino]-phenyl}-2-(4-phenylamino-piperidin-1-yl)-acetamide
N-(5-Oxo-5,6,7,8-tetrahydro-naphthalen-2-yl)-2-(4-phenylamino-piperidin-1-yl)-acetamide
N-(4-Phenoxy-phenyl)-2-(4-phenylamino-piperidin-1-yl)-acetamide
N-(4-Phenoxy-phenyl)-2-(4-{[(4-phenoxy-phenylcarbamoyl)-methyl]-phenyl-amino}-piperidin-1-yl)-acetamide
2-(4-{(4-Hydroxy-phenyl)-[(9-oxo-9H-fluoren-3-ylcarbamoyl)-methyl]-amino}-piperidin-1-yl)-N-(9-oxo-9H-fluoren-3-yl)-acetamide
optionally in form of a salt, preferably a physiologically acceptable salt, more preferably in form of a physiologically acceptable acid addition salt, most preferably a hydrochloride salt, or a corresponding solvate.

28. Process for the preparation of phenylamino-substituted piperidine compounds according to claims 1-27, **characterized in that** at least one compound of general formula (II), 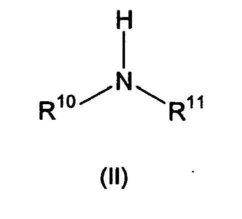 wherein R¹⁰ and R¹¹ have the meaning according to claim 1 is reacted with at least one compound of general formula (III), 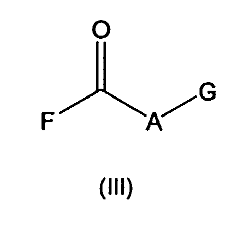 wherein A has the meaning according to claim 1, F represents halogen, hydroxy or an O-acyl group and G represents halogen, preferably chlorine, in a suitable reaction medium and preferably in the presence of at least one base and/or at least one auxiliary agent, and reacting the so obtained compound of general (IV) 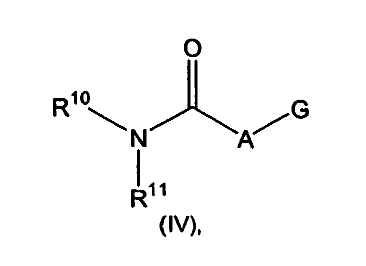 wherein A, G, R¹⁰ and R¹¹ have the above defined meaning, with at least one piperidine compound of general formula (V) and/or a salt, preferably hydrochloride, thereof, 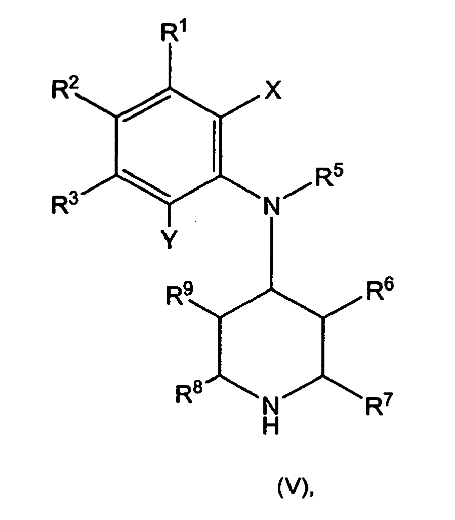 wherein R¹ to R³, R⁵ to R⁹, X and Y have the meaning according to claim 1, in a suitable reaction medium, optionally in the presence of at least one base and/or at least one auxiliary agent.

29. Process for the preparation of a physiologically acceptable salt of the phenylamino-substituted piperidine compounds according to claims 1-27, **characterized in that** at least one compound of general formula (I) having at least one basic group is reacted with at least one acid, preferably an inorganic or organic acid, preferably in the presence of a suitable reaction medium.

30. Process for the preparation of a physiologically acceptable salt of the phenylamino-substituted piperidine compounds according to claims 1-27, **characterized in that** at least one compound of general formula (I) having at least one acidic group is reacted with at least one base, preferably in the presence of a suitable reaction medium.

31. Medicament comprising at least one phenylamino-substituted piperidine compound according to claims 1-12, optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, and optionally one or more pharmaceutically acceptable adjuvants.

32. Medicament comprising at least one phenylamino-substituted piperidine compound according to claims 13-20, optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, and optionally one or more pharmaceutically acceptable adjuvants.

33. Medicament comprising at least one phenylamino-substituted piperidine compound according to claims 21-27, optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, and optionally one or more pharmaceutically acceptable adjuvants.

34. Use of at least one phenylamino-substituted piperidine compound according to claims 1-27, optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, for the manufacture of a medicament for the regulation of neuropeptide Y receptors, preferably of neuropeptide Y 5 (NPY5) receptor.

35. Use of at least one phenylamino-substituted piperidine compound according to claims 1-27, optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, for the manufacture of a medicament for the regulation of appetite or body weight.

36. Use of at least one phenylamino-substituted piperidine compound according to claims 1-27, optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, for the manufacture of a medicament for the prophylaxis or treatment of disorders of food ingestion, preferably obesity, anorexia, bulimia, cachexia or type II diabetes.

37. Use of at least one phenylamino-substituted piperidine compound according to claims 1-27, optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, for the manufacture of a medicament for the prophylaxis and/or treatment of disorders of the peripheral nervous system.

38. Use of at least one phenylamino-substituted piperidine compound according to claims 1-27, optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, for the manufacture of a medicament for the prophylaxis and/or treatment of disorders of the central nervous system.

39. Use of at least one phenylamino-substituted piperidine compound according to claims 1-27, optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, for the manufacture of a medicament for the prophylaxis or treatment of anxiety; depression or bipolar disorder; cognitive disorders, preferably memory disorders, cardiovascular diseases; pain, including neuropathic pain, chronic pain, acute pain and visceral pain; hypertensive syndrome, inflammatory diseases, immune diseases, diabetes, epilepsy, arthritis.
